(19) Europäisches Patentamt
European Patent Office
Office européen des brevets

(11) EP 1 545 442 B1

(12) # FASCICULE DE BREVET EUROPEEN

(45) Date de publication et mention de la délivrance du brevet:
**10.11.2010 Bulletin 2010/45**

(21) Numéro de dépôt: **03798231.1**

(22) Date de dépôt: **26.09.2003**

(51) Int Cl.:
***A61K 8/90*** (2006.01) ***A61K 8/81*** (2006.01)
***A61Q 1/02*** (2006.01) ***A61Q 1/04*** (2006.01)
***A61Q 1/06*** (2006.01) ***A61Q 1/10*** (2006.01)
***A61Q 3/02*** (2006.01)

(86) Numéro de dépôt international:
**PCT/FR2003/002849**

(87) Numéro de publication internationale:
**WO 2004/028487 (08.04.2004 Gazette 2004/15)**

(54) **COMPOSITION COMPRENANT UN POLYMERE SEQUENCE ET UN AGENT FILMOGENE**
ZUSAMMENSETZUNG MIT EINEM BLOCKPOLYMER UND EINEM FILMBILDENDEN MITTEL
COMPOSITION COMPRISING A BLOCK POLYMER AND A FILM-FORMING AGENT

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211949**
**20.12.2002 FR 0216437**
**21.05.2003 FR 0306121**

(43) Date de publication de la demande:
**29.06.2005 Bulletin 2005/26**

(73) Titulaire: **L'Oréal**
**75008 Paris (FR)**

(72) Inventeurs:
- **BLIN, Xavier**
**F-75015 Paris (FR)**
- **FERRARI, Veronique**
**94700 Maison-Alfort (FR)**
- **DE LA POTERIE, Valérie**
**F-77820 Le Chatelet en Brie (FR)**

(74) Mandataire: **Kromer, Christophe**
**L'Oréal**
**D.I.P.I.**
**25-29 Quai Aulagnier**
**92600 Asnières (FR)**

(56) Documents cités:
**WO-A-98/31329**
**DE-A- 10 029 697**
**FR-A- 2 798 061**
**FR-A- 2 809 306**
**FR-A- 2 832 719**
**US-A- 6 153 206**
**US-B2- 6 423 306**

EP 1 545 442 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention se rapporte à une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable contenant un polymère séquencé filmogène associé à un autre agent filmogène.

La composition peut être une poudre libre ou compactée, un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

Les compositions connues présentent une mauvaise tenue dans le temps et en particulier une mauvaise tenue de la couleur. Cette mauvaise tenue se caractérise par une modification de la couleur (virage, palissement) généralement par suite d'une interaction avec le sébum et/ou la sueur sécrétés par la peau dans le cas de fond de teint et de fard ou d'une interaction avec la salive dans le cas des rouges à lèvres. Ceci oblige l'utilisateur à se remaquiller très souvent, ce qui peut constituer une perte de temps.

Une amélioration de la tenue, en particulier de la tenue des rouges à lèvres peut être obtenue en associant une huile volatile à un polymère filmogène, comme les résines de silicone. Cependant, les propriétés de tenue obtenues restent inférieures aux attentes des consommateurs.

Il subsiste un besoin d'un produit cosmétique qui conduise à un dépôt sur les matières kératiniques, en particulier un maquillage, de bonne tenue.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

De façon surprenante, les inventeurs ont trouvé qu'en associant un polymère séquencé particulier à un agent filmogène connu, on obtient des compositions cosmétiques qui conduisent à des dépôts sur les matières kératiniques de tenue supérieure aux compositions classiques contenant des filmogènes.

De façon plus précise, l'invention a pour objet une composition cosmétique contenant un milieu liquide organique, au moins un polymère éthylénique séquencé linéaire filmogène et au moins un autre agent filmogène,

ledit polymère séquencé contenant des première et deuxième séquences reliées entre elles par un segment intermédiaire statistique comprenant au moins un monomère constitutif de la première séquence et au moins un monomère constitutif de la deuxième séquence,

la première séquence du polymère étant choisie parmi:

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence;

et ledit polymère séquencé ayant un indice de polydispersité I supérieur à 2,8.

En particulier, l'invention a pour objet une composition cosmétique contenant un milieu liquide organique, au moins un polymère éthylénique séquencé linéaire filmogène tel que décrit précédemment et au moins un autre agent filmogène soluble dans le milieu liquide organique.

L'invention a pour autre objet une composition cosmétique contenant un milieu liquide organique, au moins un polymère éthylénique séquencé linéaire filmogène tel que décrit précédemment et au moins un autre agent filmogène hydrosoluble.

L'invention a encore pour autre objet une composition cosmétique contenant un milieu liquide organique, au moins un polymère éthylénique séquencé linéaire filmogène tel que décrit précédemment et au moins une dispersion aqueuse de particules de polymère filmogène.

L'invention a encore pour autre objet une composition cosmétique contenant un milieu liquide organique, au moins un polymère éthylénique séquencé linéaire filmogène tel que décrit précédemment et au moins une dispersion non aqueuse de particules de polymère filmogène.

Le brevet US 6153206 décrit une composition cosmétique comprenant un polymère contenant des unités répétitives ayant une Tg allant de -10 à 75 °C et des unités répétitives ayant une Tg allant de 76 à 120 °C. Il décrit également des polymères blocs comprenant des blocs de poly méthyl méthacrylate et des blocs de polyisobutyl méthacrylate, et également des polymères triblocs comprenant des blocs de poly méthyl méthacrylate, des blocs de polyisobutyl méthacrylate et des blocs d'éthyl methacrylate.

Le polymère éthylénique séquencé linéaire filmogène est avantageusement non élastomère. Le polymère éthylénique séquencé linéaire filmogène est avantageusement exempt de motif styrène.

L'invention se rapporte aussi à un procédé de maquillage de la peau et/ou des lèvres et/ou des phanères consistant à appliquer sur la peau et/ou les lèvres et/ou les phanères la composition tel que définie précédemment.
La composition selon l'invention peut être appliquée sur la peau aussi bien du visage que du cuir chevelu et du corps, des muqueuses comme les lèvres, de l'intérieur des paupières inférieures, et des phanères comme les ongles, les cils, les cheveux, les sourcils, voire les poils.
De préférence, la composition selon l'invention est une composition non rincée.
L'invention se rapporte également à l'utilisation cosmétique de la composition définie ci-dessus pour améliorer la tenue du maquillage sur la peau et/ou les lèvres et/ou les phanères.
En particulier, dans le cas d'une composition de revêtement des cils ou mascara, une telle composition permet l'obtention, après application sur les cils, d'un film de maquillage présentant une bonne tenue , en particulier à l'eau, lors de baignades ou de douches par exemple, aux frottements, notamment des doigts et/ou bien encore aux larmes, à la sueur ou au sébum.
L'invention a enfin pour objet l'utilisation d'un agent filmogène dans une composition contenant un polymère séquencé tel que décrit précédemment pour obtenir une composition de bonne texture, facile à appliquer et conduisant sur les lèvres et/ou les phanères à un dépôt de bonne tenue.

**Polymère séquencé :**

La composition selon la présente invention contient au moins un polymère séquencé. Par polymère "séquencé", on entend un polymère comprenant au moins 2 séquences distinctes, de préférence au moins 3 séquences distinctes.
Selon un mode de réalisation, le polymère séquencé de la composition selon l'invention est un polymère éthylénique. Par polymère "éthylénique", on entend un polymère obtenu par polymérisation de monomères comprenant une insaturation éthylénique.
Selon un mode de réalisation, le polymère séquencé de la composition selon l'invention est un polymère linéaire. Par opposition, un polymère à structure non linéaire est, par exemple, un polymère à structure ramifiée, en étoile, greffée, ou autre.
Selon un mode de réalisation, le polymère séquencé de la composition selon l'invention est un polymère filmogène. Par polymère "filmogène", on entend un polymère apte à former à lui seul ou en présence d'un agent auxiliaire de filmification, un film continu et adhérent sur un support, notamment sur les matières kératiniques.
Selon un mode de réalisation, le polymère séquencé de la composition selon l'invention est un polymère non élastomère.
Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.
De manière plus spécifique, par "polymère non élastomère" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50 %.
Plus précisément, le caractère non élastomère du polymère est déterminé selon le protocole suivant :

> On prépare un film de polymère par coulage d'une solution du polymère dans une matrice téflonnée puis séchage pendant 7 jours dans une ambiance contrôlée à 23±5°C et 50±10 % d'humidité relative.

On obtient alors un film d'environ 100 µm d'épaisseur dans lequel sont découpées des éprouvettes rectangulaires (par exemple à l'emporte-pièce) d'une largeur de 15 mm et d'une longueur de 80 mm.
On impose à cet échantillon une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($I_0$) de l'éprouvette.
On détermine la recouvrance instantanée Ri de la manière suivante :

- on étire l'éprouvette de 30 % ($\varepsilon_{max}$) c'est-à-dire environ 0,3 fois sa longueur initiale ($I_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.
La recouvrance retardée en % ($R_{2h}$) est donnée par la formule ci-après:

$$R_{2h}= (\varepsilon_{max} - \varepsilon_{2h})/\varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.
Selon un autre mode de réalisation, le polymère séquencé de la composition selon l'invention ne comprend pas de motif styrène. Par polymère exempt de motif styrène, on entend un polymère comprenant moins de 10%, de préférence moins de 5%, de préférence moins de 2%, de préférence encore moins de 1% en poids i) de motif styrène de formule $-CH(C_6H_5)-CH_2-$ ou ii) de motif styrène substitué, comme par exemple le méthylstyrène, le chlorostyrène ou le chlorométhylstyrène.
Selon un mode de réalisation, le polymère séquencé de la composition selon l'invention est issu de monomères éthyléniques aliphatiques. Par monomère aliphatique, on entend un monomère ne comprenant aucun groupe aromatique.
Selon un mode de réalisation, le polymère séquençé est un polymère éthylénique issu de monomères éthyléniques aliphatiques comprenant une double liaison carbone carbone et au moins un groupement ester -COO- ou amide -CON-. Le groupe ester peut être lié à un des deux carbones insaturés par l'atome de carbone ou l'atome d'oxygène. Le groupe amide peut être lié à un des deux carbones insaturés par l'atome de carbone ou l'atome d'azote.
Selon un mode de mise en oeuvre, le polymère séquencé comprend au moins une première séquence et au moins une deuxième séquence telles que décrites précédemment.
Par "au moins" une séquence, on entend une ou plusieurs séquences.
On précise que dans ce qui précède et ce qui suit les termes "première" et "deuxième" séquences ne conditionnent nullement l'ordre desdites séquences (ou blocs) dans la structure du polymère.
Selon un mode de mise en oeuvre, le polymère séquencé comprend au moins une première séquence et au moins une deuxième séquence ayant des températures de transition vitreuse (Tg) différentes.
Dans ce mode de mise en oeuvre, les première et deuxième séquences peuvent être reliées entre elles par un segment intermédiaire ayant une température de transition vitreuse comprise entre les températures de transition vitreuse des première et deuxième séquences.
De préférence, la séquence intermédiaire est issue essentiellement de monomères constitutifs de la première séquence et de la deuxième séquence.
Par "essentiellement", on entend au moins à 85%, de préférence au moins à 90%, mieux à 95% et encore mieux à 100%.
Selon un mode de mise en oeuvre, le polymère séquencé comprend au moins une première séquence et au moins une deuxième séquence incompatibles dans le milieu liquide organique de la composition de l'invention.
Par "séquences incompatibles l'une avec l'autre", on entend que le mélange formé du polymère correspondant à la première séquence et du polymère correspondant à la deuxième séquence, n'est pas miscible dans le liquide organique majoritaire en poids contenu dans le milieu liquide organique de la composition, à température ambiante (25°C) et pression atmosphérique ($10^5$ Pa), pour une teneur du mélange de polymères supérieure ou égale à 5 % en poids, par rapport au poids total du mélange (polymères et liquide organique majoritaire), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas où le milieu liquide organique comprend un mélange de liquides organiques, dans l'hypothèse de deux ou plusieurs liquides présents en proportions massiques identiques, ledit mélange de polymères est non miscible dans au moins l'un d'entre eux.
Dans le cas où le milieu liquide organique comprend un seul liquide organique, ce dernier constitue bien évidemment le liquide majoritaire en poids.
Par "milieu liquide organique", on entend un milieu contenant au moins un liquide organique, c'est-à-dire, au

moins un composé organique liquide à température ambiante (25°C) et pression atmosphérique ($10^5$ Pa). Selon un mode de mise en oeuvre, le liquide majoritaire du milieu liquide organique est une huile (corps gras) volatile ou non volatile. De préférence, le liquide organique est cosmétiquement acceptable (tolérance, toxicologie et toucher acceptables). Le milieu liquide organique est cosmétiquement acceptable, en ce sens qu'il est compatible avec les matières kératiniques, comme les huiles ou les solvants organiques couramment employés dans les compositions cosmétiques.

Selon un mode de mise en oeuvre, le liquide majoritaire du milieu liquide organique est le solvant ou un des solvants de polymérisation du polymère séquencé tels qu'ils sont décrits ci-après.

Par solvant de polymérisation, on entend un solvant ou un mélange de solvants. Le solvant de polymérisation peut être choisi notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol, ou l'isododécane.

De manière générale, le polymère séquencé peut être incorporé dans la composition à une teneur élevée en matières sèche, typiquement supérieure à 10%, supérieure à 20% et de préférence encore supérieure à 30% et de préférence encore supérieure à 45% en poids par rapport au poids total de la composition tout en étant faciles à formuler.

De façon préférentielle, le polymère séquencé ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

Selon un mode de mise en oeuvre, le polymère séquencé a un indice de polydispersité I supérieur à 2,8

Avantageusement, le polymère séquencé utilisé dans les compositions selon l'invention a un indice de polydispersity I allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère séquencé est de préférence inférieure ou égale à 300 000, elle va par exemple de 35 000 à 200 000, et mieux de 45 000 à 150 000.

La masse moyenne en nombre (Mn) du polymère séquencé est de préférence inférieure ou égale à 70 000, elle va par exemple de 10 000 à 60 000, et mieux de 12 000 à 50 000.

Chaque séquence ou bloc du polymère séquencé est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi_i / Tg_i) ,$$

$\omega_i$ étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

a) Séquence avant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C .

La séquence ayant une Tg supérieure ou égale à 40°C peut être un homopolymère ou un copolymère.

La séquence ayant une Tg supérieure ou égale à 40°C peut être issue en totalité ou en partie de un ou plusieurs monomères, qui sont tels que l'homopolymère préparé à partir de ces monomères a une températures de transition vitreuse supérieure ou égale à 40°C.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin, .

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,

- les acrylates de formule $CH_2 = CH\text{-}COOR_2$
  dans laquelle $R_2$ représente un groupe cycloalkyl en $C_4$ à $C_{12}$ tel que l'acrylate d'isobornyle ou un groupe tertio butyle,

- les (méth)acrylamides de formule :

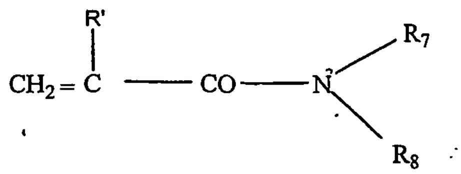

  où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
  et R' désigne H ou méthyle. Comme exemple de monomères, on peut citer le N-butylacrylamide, le N-t-butylacrylamide, le N-isopropytacrytamide, le N,N-diméthylacrylamide et le N,N-dibutylacrylamide ,

- et leurs mélanges.

Des monomères principaux particulièrement préférés sont le méthacrylate de méthyle, le (méth)acrylate d'isobutyle, le (méth)acrylate d'isobornyle et leurs mélanges.

b) Séquence ayant une Tg inférieure ou égale à 20°C

La séquence ayant une Tg inférieure ou égale à 20°C a par exemple une Tg allant de - 100 à 20°C, de préférence inférieure ou égale à 15°C, notamment allant de -80°C à 15°C et mieux inférieure ou égale à 10°C, par exemple allant de -50°C à 0°C.
La séquence ayant une Tg inférieure ou égale à 20°C peut être un homopolymère ou un copolymère.
La séquence ayant une Tg inférieure ou égale à 20°C peut être issue en totalité ou en partie de un ou plusieurs monomères, qui sont tel(s) que l'homopolymère préparé à partir de ces monomères a une température de transition vitreuse inférieure ou égale à 20°C.
Dans le cas où cette séquence est un homopolymère, elle est issue de monomères, qui sont tel(s) que les homopolmères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).
Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.
Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-CO-O-CH} = CH_2$
  où $R_5$ représente un groupe alkyle en $C_4$ à $C_{12}$ linéaire ou ramifié ;

- les éthers de vinyle et d'alkyle en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

c) Séquence ayant une Tg comprise entre 20 et 40°C

La séquence qui a une Tg comprise entre 20 et 40°C peut être un homopolymère ou un copolymère.
La séquence ayant une Tg comprise entre 20 et 40°C peut être issue en totalité ou en partie de un ou de plusieurs monomères, qui sont tel(s) que l'homopolymère préparé à partir de ces monomères a une température de transition vitreuse comprise entre 20 et 40°C.
La séquence ayant une Tg comprise entre 20 et 40°C peut être issue en totalité ou en partie de monomères

qui sont tel(s) que l'homopolymère correspondant a une Tg supérieure ou égale à 40°C et de monomères qui sont tel (s) que l'homopolymère correspondant a une Tg inférieure ou égale à 20°C.

Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).

Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.
Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et/ou
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,
  lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.

De préférence, la proportion de la deuxième séquence ayant une Tg inférieure ou égale à 20°C va de 10 à 85% en poids du polymère, mieux de 20 à 70% et encore mieux de 20 à 50%.

De préférence, chacune des première et deuxième séquences comprend au moins un monomère choisi parmi l'acide acrylique, les esters d'acide acrylique, l'acide (méth)acrylique, les esters d'acide (méth)acrylique et leurs mélanges.

Avantageusement, chacune des première et deuxième séquences est issue en totalité d'au moins un monomère choisis parmi l'acide acrylique, les esters d'acide acrylique, l'acide (méth)acrylique, les esters d'acide (méth)acrylique et leurs mélanges.

Chacune des séquences peut néanmoins contenir en proportion minoritaire au moins un monomère constitutif de l'autre séquence.
Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyrjdine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs

substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,

- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy) silane,

- et leurs mélanges.

Des monomères additionnels particulièrement préférés sont l'acide acrylique, l'acide méthacrylique, le méthacrylate de trifluoroéthyle et leurs mélanges.

Selon un mode de réalisation, chacune des première et deuxième séquence du polymère séquencé comprend au moins un monomère choisi parmi les esters d'acide (méth)acrylique et éventuellement au moins un monomère additionnel tel que l'acide (méth)acrylique, et de leurs mélanges.

Selon un autre mode de réalisation, chacune des première et deuxième séquence du polymère séquencé est issue en totalité d'au moins un monomère choisi parmi les esters d'acide (méth)acrylique et éventuellement d'au moins un monomère additionnel tel que l'acide (méth)acrylique, et de leurs mélanges.

Selon un mode préféré de réalisation, le polymère séquencé est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' ( allant de 3 à 6 h) au bout duquel le mélange est ramené à température ambiante,
- on obtient le poLymère en solution dans le solvant de polymérisation.

Premier mode de réalisation

Selon un premier mode de réalisation, le polymère séquencé comprend une première séquence ayant une Tg supérieure ou égale à 40°C, telle que décrite plus haut au a) et une deuxième séquence ayant une Tg inférieure ou égale à 20°C, telle que décrite plus haut au b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C va de 20 à 90% en

poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Selon une troisième variante, le polymère selon l'invention peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à - 55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon une quatrième variante, le polymère selon l'invention peut comprendre :

- une première séquence de Tg supérieure ou égale à 40°C, par exemple allant de 85 à 115°C, qui est un copolymère acrylate d'isobornyle/méthacrylate de méthyle,
- une deuxième séquence de Tg inférieure ou égale à 20°C, par exemple allant de -85 à
- 55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère selon l'invention peut comprendre :

- une première séquence de Tg supérieure ou égale à 40°C, par exemple allant de 95 à 125°C, qui est un copolymère acrylate d'isobornyle / méthacrylate d'isobornyle;
- une deuxième séquence de Tg inférieure ou égale à 20°C, par exemple allant de -85 à
- 55°C, qui est un homopolymère d'acrylate d'éthyl-2 hexyle et
- une séquence intermédiaire qui est un copolymère statistique acrylate d'isobornyle/ méthacrylate d'isobornyle/ acrylate d'éthyl-2 hexyle.

Selon une sixième variante, le polymère selon l'invention peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique méthacrylate d'isobornyle/méthacrylate d'isobutyle/acrylate d'isobutyle.

Selon une septième variante, le polymère selon l'invention peut comprendre :

- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique acrylate d'isobornyle/méthacrylate d'isobornyle/acrylate d'isobutyle.

Selon une huitième variante, le polymère selon l'invention peut comprendre :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à
- 5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Les exemples qui suivent illustrent de manière non limitative des polymères correspondant à ce premier mode de réalisation.
Les quantités sont exprimées en gramme.

**<u>Exemple 1 : Préparation d'un polymère de poly(acrylate d'isobornyle/méthacrylate de méthyle/acrylate d'éthyl-2 hexyle</u>**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 150 g d'acrylate d'isobornyle, 60 g de méthacrylate de méthyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate de méthyle) ayant une Tg de 100°C, une deuxième séquence polyacrylate d'éthyl-2 hexyle ayant une Tg de - 70°C et une séquence intermédiaire qui est un polymère statistique acrylate d'isobornyle/méthacrylate de méthyle/acrylate d'éthyl-2 hexyle.
Ce polymère présente une masse moyenne en poids de 76 500 et une masse moyenne en nombre de 22 000, soit un indice de polydispersité I de 3,48.

**<u>Exemple 2 : Préparation d'un Polymère de poly(acrylate d'isobornyle/méthacrylate d'isobornyle /acrylate d'éthyl-2 hexyle)</u>**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobornyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.
Ce polymère présente une masse moyenne en poids de 103 900 et une masse moyenne en nombre de 21 300, soit un indice de polydispersité I de 4.89.

**Exemple 3 : Préparation d'un polymère de poly(acrylate d'isobornyle/methacrylate d'isobutyle /acrylate d'isobutyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 120 g de acrylate d'isobornyle, 90 g de méthacrylate d'isobutyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobutyle) ayant une Tg de 75°C, une deuxième séquence polyacrylate d'isobutyle ayant une Tg de - 20°C et une séquence intermédiaire qui est un polymère statistique acrylate d'isobornyle/méthacrylate d'isobutyle/acrylate d'isobutyle.
Ce polymère présente une masse moyenne en poids de 144 200 et une masse moyenne en nombre de 49 300, soit un indice de polydispersité I de 2,93.

Second mode de réalisation

Selon un second mode de réalisation, le polymère séquencé comprend une première séquence ayant une température de transition vitreuse (Tg) comprise entre 20 et 40°C, conforme aux séquences décrites au c) et une deuxième séquence ayant une température de transition vitreuse inférieure ou égale à 20°C, telle que décrite plus haut au b) ou une température de transition vitreuse supérieure ou égale à 40°C, telle que décrite au a) ci-dessus.
De préférence, la proportion de la première séquence ayant une Tg comprise entre 20 et 40°C va de 10 à 85% en poids du polymère, mieux de 30 à 80% et encore mieux de 50 à 70%.
Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.
Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.
De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.
Avantageusement, la deuxième séquence ayant une Tg inférieure ou égale à 20°C ou ayant une Tg supérieure ou égale à 40°C est un homopolymère.
Ainsi, selon première variante de ce second mode de réalisation, le polymère séquencé peut comprendre :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

Selon seconde variante de ce second mode de réalisation, le polymère séquencé peut comprendre :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à - 35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère séquencé peut comprendre :

- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg supérieure ou égale à 40°C, par exemple allant de 85 à 115°C, qui est un homopolymère d'acrylate d'isobornyle et
- une séquence intermédiaire qui est un copolymère statistique acrylate d'isobornyle / acrylate de méthyle/acide acrylique.

La composition selon l'invention contient de préférence de 0,1 à 60% en poids en matière active (ou matière séche) du polymère, de préférence de 0,5 à 50 % en poids, et de préférence encore de 1 à 40% en poids.

**Agent filmogène**

La composition de l'invention contient également au moins un agent filmogène, qui peut être un polymère organique ou minéral. L'agent filmogène, lorsqu'il est un polymère organique, n'est pas un polymère séquencé linéaire filmogène éthylénique tel que décrit ci-dessus.

Dans un mode de réalisation, le polymère organique filmogène est au moins un polymère choisi parmi le groupe comprenant :

- les polymères filmogènes solubles dans le milieu liquide organique, en particulier les polymères liposolubles, lorsque le milieu liquide organique comprend au moins une huile,
- les polymères filmogènes dispersibles dans le milieu solvant organique, en particulier les polymères sous la forme de dispersions non aqueuses de particules polymères, de préférence des dispersions dans les huiles siliconées ou hydrocarbonées ; dans un mode de réalisation, les dispersions non aqueuses de polymère comprennent des particules polymères stabilisées sur leur surface par au moins un agent stabilisant ; ces dispersions non aqueuses sont souvent appelées « NAD [non-aqueous dispersions] »,
- les dispersions aqueuses de particules de polymères filmogènes, souvent appelées « latex » ; dans ce cas, la composition doit comprendre, outre le milieu liquide organique, une phase aqueuse,
- les polymères filmogènes hydrosolubles ; dans ce cas, la composition doit comprendre, outre le milieu liquide organique, une phase aqueuse.

Dans un mode de réalisation, l'agent filmogène est un polymère organique filmogène soluble dans le milieu liquide organique.

*1/ Polymères solubles dans le milieu liquide organique*

Lorsque le milieu liquide organique de la composition comprend au moins une huile, l'agent filmogène peut être un polymère soluble dans ladite huile. Dans ce cas, on parle de polymère liposoluble. Le polymère liposoluble peut être d'un type chimique quelconque et peut être notamment choisi parmi:

a) les homopolymères et les copolymères liposolubles et amorphes des oléfines, des cyclooléfines, du butadiène, de l'isoprène, du styrène, des éthers, des esters ou amides vinyliques, des esters ou amides de l'acide (méth) acrylique contenant un groupement alkyle en $C_{4-50}$ linéaire, ramifié ou cyclique, et préférablement amorphes. Les homopolymères et les copolymères liposolubles préférés sont obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci. On citera, par exemple, le copolymère d'acrylate d'alkyle/acrylate de cycloalkyle commercialisé par PHOENIX CHEM. sous la dénomination GIOVAREZ AC-5099 ML, et les copolymères de vinylpyrrolidone, tels que les copolymères d'un alkène en $C_2$ à $C_{30}$, tel qu'en $C_3$ à $C_{22}$, et des associations de ceux-ci, peuvent être utilisés. Comme exemples de copolymères de VP pouvant être utilisés dans l'invention, on peut citer le copolymère de VP/laurate de vinyle, de VP/stéarate de vinyle, la polyvinylpyrrolidone (PVP) butylée, de VP/hexadécène, de VP/triacontène ou de VP/acide acrylique/méthacrylate de lauryle.
Comme copolymères liposolubles particuliers, on peut citer :

i) les polymères de silicone-acrylique greffés ayant un squelette siliconé, des greffons acryliques ou ayant un squelette acrylique, les greffons de silicone tels que le produit commercialisé sous la dénomination SA 70.5 par 3M et décrit dans les brevets US 5 725 882, US 5 209 924, US 4 972 037, US 4 981 903, US 4 981 902, US 5 468 477, et dans les brevets US 5 219 560 et EP 0 388 582.

ii) les polymères liposolubles portant des groupements fluorés appartenant à l'une des classes décrites dans le texte ci-dessus, en particulier ceux décrits dans le brevet US 5 948 393, les copolymères de (méth)acrylate d'alkyle/(méth)acrylate de perfluoroalkyle décrits dans les brevets EP 0 815 836 et US 5 849 318.

iii)les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.

Dans un mode de réalisation, l'agent filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/ propylène (EP), un bloc butadiène (B), un bloç isoprène (I), un bloc acrylate (A), un bloc méthacrylate (MA) ou une association de ces blocs. Le copolymère comprenant au moins un bloc constitué de motifs styrène ou dérivés du styrène peut être un copolymère tribloc, et en particulier du type polystyrène/polyisoprène ou polystyrène/polybutadiène, tels que ceux commercialisés ou fabriqués sous la dénomination « Luvitol HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés. Des copolymères de styrène-méthacrylate peuvent également être utilisés.
copolymère comprenant au moins un bloc constitué de motifs styrène ou dérivés du styrène peut être, par exemple, le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS), le Gelled Permethyl 99A-750, le Gelled Permethyl 99A-753-58 (mélange de polymère bloc en étoile et de polymère tribloc), le Gelled Permethyl 99A-753-59 (mélange de polymère bloc en étoile et de polymère tribloc), le Versagel 5970 et le Versagel 5960 de chez Penreco (mélange de polymère en étoile et de polymère tribloc dans l'isododécane), et OS 129880, OS 129881 et OS 84383 de chez Lubrizol (copolymère de styrène-méthacrylate).

Dans un mode de réalisation, l'agent filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).
Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.
Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.
Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.
De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth) acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000. Comme exemples de polymères liposolubles pouvant être utilisés dans l'invention, on peut citer les polyalkylènes, les copolymères d'alcènes en $C_2$-$C_{20}$, en particulier le polybutène.

b) les polycondensats amorphes et liposolubles, de préférence ne comprenant pas de groupements donneurs d'interactions hydrogène, en particulier les polyesters aliphatiques ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, solides à température ambiante tel que défini dans la demande de brevet FR 0 113 920, non encore publiée.

c) les polysaccharides amorphes et liposolubles comprenant des chaînes latérales alkyl (éther ou ester), en particulier les alkylcelluloses ayant un radical alkyle en $C_1$ à $C_8$ saturé ou insaturé, linéaire ou ramifié, tel que l'éthylcellulose et la propylcellulose.
Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.
Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

d) les résines silicones solubles ou gonflables par des huiles de silicone. Ces résines sont des polyorganosiloxanes partiellement réticulés qui, en fonction du taux de réticulation, seront solubles ou gonflables par les huiles de silicone de la phase huileuse du milieu liquide organique. Ces résines silicones peuvent choisies parmi la liste non limitative suivante : résines MQ ou triméthylsiloxysilicates, polysilesquioxane ou polymères réticulés de diméthicone/ vinyldiméthicone.

*II/ Dispersions non aqueuses de particules polymères*

La composition peut contenir un agent filmogène choisi parmi les dispersions non aqueuses de particules polymères. Les particules sont généralement sphériques. Avant leur incorporation dans la composition de l'invention, les particules sont généralement dispersées dans une phase grasse liquide physiologiquement acceptable, telle que les huiles hydrocarbonées ou les huiles siliconées. Selon un mode de mise en oeuvre, Ces dispersions sont généralement connues comme « NAD » (dispersions non aqueuses) de polymère, par opposition aux réseaux, qui sont des dispersions aqueuses de polymère. Ces dispersions peuvent être notamment sous la forme de nanoparticules de polymères en dispersion stable dans ladite phase grasse. Dans un mode de réalisation, les nanoparticules ont une taille comprise entre 5 nm et 600 nm. Cependant, il est possible d'obtenir des particules polymères d'une taille allant jusqu'à 1 $\mu$m.

L'un des avantages de la dispersion de polymère de la composition de l'invention est la possibilité de varier la température de transition vitreuse (Tg) du polymère ou du système de polymère (polymère plus additif du type plastifiant), et donc de passer d'un polymère dur à un polymère plus ou moins mou, rendant possible l'ajustement des propriétés mécaniques de la composition, selon l'application prévue et en particulier selon le film déposé.

Les polymères en dispersion pouvant être utilisés dans la composition de l'invention ont préférablement un poids moléculaire allant d'environ 2000 à 10 000 000, et une Tg allant de -100°C à 300°C, et mieux encore de -50°C à 50°C, et préférablement de -10°C à 100°C.

Il est possible d'utiliser des polymères filmogènes, ayant préférablement une faible Tg, inférieure ou égale à la température de la peau et notamment inférieure ou égale à 40°C. On obtient ainsi une dispersion, pouvant former un film lors de son application à un support.

On peut citer parmi les polymères filmogènes les homopolymères ou les copolymères acryliques ou vinyliques radicalaires, ayant préférablement une Tg inférieure ou égale à 40°C et notamment allant de -10°C à 30°C, utilisés seuls ou en mélange.

Par l'expression « polymère radicalaire », on entend un polymère obtenu par la polymérisation de monomères contenant une insaturation, notamment une insaturation éthylénique, chaque monomère étant capable d'une homopolymérisation (au contraire des polycondensats). Les polymères radicalaires peuvent notamment être des polymères ou

des copolymères vinyliques, notamment des polymères acryliques.

Les polymères vinyliques peuvent résulter de la polymérisation de monomères éthylèniquement insaturés contenant au moins un groupement acide et/ou d'esters de ces monomères acides et/ou d'amides de ces acides.

Comme monomères portant un groupement acide, il est possible d'utiliser des acides carboxyliques insaturés $\alpha$,$\beta$-éthyléniques, tels que l'acide acrylique, l'acide méthacrylique, l'acide crotonique, l'acide maléique ou l'acide itaconique. L'acide (méth)acrylique et l'acide crotonique sont utilisés de préférence, et plus préférentiellement l'acide (méth) acrylique.

Les esters de monomères acides sont choisis de manière avantageuse parmi les esters de l'acide (méth) acrylique (également connus comme (méth)acrylates), par exemple les (méth)acrylates d'alkyle, en particulier d'un alkyle en $C_1$-$C_{20}$ et préférablement en $C_1$-$C_6$, les (méth)acrylates d'aryle, en particulier d'un aryle en $C_6$-$C_{10}$, et les (méth)acrylates d'hydroxyalkyle, en particulier d'un hydroxyalkyle en $C_2$-$C_6$. Les (méth)acrylates d'alkyle pouvant être cités comportent le (méth)acrylate de méthyle, d'éthyle, de butyle, d'isobutyle, de 2-éthylhexyle et de lauryle. Les (méth) acrylates d'hydroxyalkyle pouvant être cités comportent le (méth)acrylate d'hydroxyéthyle et le (méth)acrylate de 2-hydroxypropyle. Les (méth)acrylates d'aryle pouvant être cités comportent l'acrylate de benzyle ou de phényle.

Les esters de l'acide (méth)acrylique étant particulièrement préférés sont les (méth)acrylates d'alkyle.

Les polymères radicalaires qui sont de préférence utilisés sont les copolymères de l'acide (méth)acrylique et d'un (méth)acrylate d'alkyle, notamment d'un alkyle en $C_1$-$C_4$. Plus préférentiellement, on peut utiliser les acrylates de méthyle, éventuellement copolymérisés avec l'acide acrylique.

Les amides des monomères acides pouvant être cités comportent les (méth)acrylamides, et notamment les N-alkyl(méth)acrylamides, en particulier d'un alkyle en $C_2$-$C_{12}$, tel que le N-éthylacrylamide, le N-t-butylacrylamide et le N-octylacrylamide, les N-dialkyl($C_1$-$C_4$)(méth)acrylamides.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha$.$\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle. Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogènè du groupement alkyle sont substitués par des atomes de fluor.

Comme amides des monomères acides, on peut par exemple citer les (méth)acrylamides, et notamment les N-alkyl (méth)acylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle. Comme monomères styréniques, on peut citer le styrène et l'alpha-méthyl styrène.

De manière non limitative, les polymères en dispersion de l'invention peuvent être choisis parmi les polymères ou les copolymères suivants : les polyuréthanes, les polyuréthane-acryliques, les polyurées, les polyurée-polyuréthanes, les polyester-polyuréthanes, les polyéther-polyuréthanes, les polyesters, les polyesteramides, les polyesters à chaîne grasse, les alkydes ; les polymères ou les copolymères acryliques et/ou vinyliques ; les copolymères acrylique-silicone ; les polyacrylamides ; les polymères siliconés, par exemple les polyuréthanes siliconés ou les acryliques siliconés, et les polymères fluorés et des mélanges de ceux-ci.

Le ou les polymère(s) en dispersion huileuse peut(peuvent) représenter (en matière sèche ou matière active) de 0,1% à 60% du poids de la composition, préférablement de 2% à 40% et mieux encore de 4% à 25%. Pour un stabilisant qui est solide à température ambiante, la quantité de matière sèche dans la dispersion représente la quantité

totale de polymère et de stabilisant.

Les polymères liposolubles ou dispersibles dans la composition de l'invention peuvent également être utilisés en une quantité allant de 0,01% à 20% (en matière active) par rapport au poids total de la composition, tel que par exemple de 1% à 10%, le cas échéant.

*III) Dispersions aqueuses de particules polymères*

Selon un autre mode de réalisation, le polymère filmogène peut être choisi parmi les dispersions aqueuses de particules polymères, dans le cas où la composition selon l'invention comprend une phase aqueuse.

La dispersion aqueuse comprenant un ou plusieurs polymères filmogènes peut être préparée par l'homme de l'art sur la base de ses connaissances générales, en particulier par une polymérisation en émulsion ou par une mise en dispersion du polymère précédemment formé.

Parmi les polymères filmogènes pouvant être utilisés dans la composition selon la présente invention, on peut citer les polymères synthétiques du type polycondensat ou du type radical, les polymères d'origine naturelle, et des mélanges de ceux-ci.

Parmi les polycondensats, on peut également citer les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino), comprenant en outre soit un groupement carboxylate ou acide carboxylique, soit un groupement sulfonate ou acide sulfonique, voire un groupement amine tertiaire neutralisable ou un groupement ammonium quaternaire.

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques. Le glycérol, le pentaérythritol, le sorbitol et le triméthylolpropane peuvent être utilisés comme polyols.

Les polyesteramides peuvent être obtenus de manière analogue aux polyesters, au moyen de la polycondensation de diacides avec des diamines ou des aminoalcools. L'éthylènediamine, l'hexaméthylènediamine, et la méta- ou para-phénylènediamine peuvent être utilisées comme diamine. La monoéthanolamine peut être utilisée comme aminoalcool.

Comme monomère portant un groupement anionique pouvant être utilisé pendant la polycondensation, on peut citer par exemple, l'acide diméthylolpropionique, l'acide trimellitique ou un dérivé tel que l'anhydride trimellitique, le sel de sodium de l'acide 3-sulfopentanediol et le sel de sodium de l'acide 5-sulfo-1,3-benzènedicarboxylique. Les polyesters ayant une chaîne grasse peuvent être obtenus par l'intermédiaire de l'utilisation de diols ayant une chaîne grasse lors de la polycondensation. Les résines d'époxyester peuvent être obtenues par la polycondensation d'acides gras avec un condensat au niveau des extrémités $\alpha,\omega$-diépoxy.

Les polymères radicalaires peuvent être en particulier les polymères ou les copolymères acryliques et/ou vinyliques. Les polymères à radical anionique sont préférés. Comme monomère portant un groupement anionique pouvant être utilisé lors de la polymérisation radicalaire, on peut citer l'acide acrylique, l'acide méthacrylique, l'acide crotonique, l'anhydride maléique et l'acide 2-acrylamido-2-méthylpropanesulfonique.

Les polymères acryliques peuvent résulter de la copolymérisation de monomères choisis parmi les esters et/ou les amides de l'acide acrylique ou de l'acide méthacrylique. Comme exemples de monomères du type ester, on peut

citer le méthacrylate de méthyle, le méthacrylate d'éthyle, le méthacrylate de butyle, le méthacrylate d'isobutyle, le méthacrylate de 2-éthylhexyle et le méthacrylate de lauryle. Comme exemples de monomères du type amide, on peut citer le N-t-butylacrylamide et le N-t-octylacrylamide.

On utilise de préférence les polymères acryliques obtenus par la copolymérisation de monomères à insaturation éthylénique contenant des groupements hydrophiles, préférablement de nature non ionique, tels que l'acrylate d'hydroxyéthyle, l'acrylate de 2-hydroxypropyle, le méthacrylate d'hydroxyéthyle et le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemples d'esters vinyliques, on peut citer l'acétate de vinyle, le néodécanoate de vinyle, le pivalate de vinyle, le benzoate de vinyle et le t-butylbenzoate de vinyle.

On peut également utiliser des copolymères d'acrylique/silicone, voire des copolymères de nitrocellulose/ acrylique.

Les polymères d'origine naturelle, éventuellement modifiés, peuvent être choisis parmi la gomme-laque, la gomme sandaraque, les dammars, l'élémis, les copals, les' dérivés cellulosiques, et des mélanges de ceux-ci.

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

Lorsqu'une dispersion aqueuse de particules polymères est utilisée, la teneur en matière sèche de ladite dispersion aqueuse peut être de l'ordre de 5-60% en poids, et préférablement de 30-50%.

La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » ou « Daitosol 5000 SJ » par la société DAITO KASEY KOGYO; « Syntran 5760 » par la société Interpolymer ou encore les dispersions aqueuses de polyuréthane vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, «Aquamere H-1511® » par la société HYDROMER ; les sulfopolyesters vendus sous le nom de marque « Eastman AQ®» par la société EASTMAN CHEMICAL PRODUCTS, les dispersions vinyliques comme le « Mexomère PAM » et leurs mélanges.

*IV) Polymères hydrosolubles*

Dans le cas où la composition comprend une phase aqueuse, le polymère filmogène peut être un polymère hydrosoluble. Le polymère hydrosoluble est donc solubilisé dans la phase aqueuse de la composition.

Parmi les polymères filmogènes hydrosolubles, on peut citer les polymères cationiques suivants :

(1) les polymères ou copolymères acryliques, tels que les polyacrylates ou les polyméthacrylates ; les copolymères de la famille (1) peuvent contenir en outre un ou plusieurs motifs dérivant de comonomères pouvant être choisis parmi la famille des acrylamides, des méthacrylamides, des diacétoneacrylamides, des acrylamides et méthacrylamides substitués sur l'azote par des alkyles inférieurs, des acides acrylique ou méthacrylique ou des esters de ceux-ci, des vinyllactames tels que la vinylpyrrolidone ou le vinylcaprolactame, des esters vinyliques.

Ainsi, parmi ces copolymères de la famille (1), on peut citer :

- les copolymères d'acrylamide et de méthacrylate de diméthylaminoéthyle quaternisés par le sulfate de diméthyle, ou par un halogénure de diméthyle tel que celui commercialisé sous la dénomination HERCOFLOC par la société HERCULES,
- le copolymère d'acrylamide et de chlorure de méthacryloyloxyéthyltriméthylammonium décrit, par exemple, dans la demande de brevet EP-A-0 809 76 et commercialisé sous la dénomination BINA QUAT P 100 par la société CIBA GEIGY,
- le copolymère d'acrylamide et de méthosulfate de méthacryloyloxyéthyltriméthylammonium commercialisé sous la dénomination RETEN par la société HERCULES,
- les copolymères de vinylpyrrolidone/acrylate ou méthacrylate de dialkylaminoalkyle, quaternisés ou non quaternisés, tels que les produits commercialisés sous la dénomination « GAFQUAT » par la société ISP, tels que par exemple « GAFQUAT 734 » ou « GAFQUAT 755 », ou bien les produits désignés par « COPOLYMER 845, 958 et 937 ». Ces polymères sont décrits en détail dans les brevets français 2 077 143 et 2 393 573,

- les terpolymères de méthacrylate de diméthylaminoéthyle/vinylcaprolactame/vinylpyrrolidone tels que le produit commercialisé sous la dénomination GAFFIX VC 713 par la société ISP, et
- le copolymère de vinylpyrrolidone/diméthylaminopropylméthacrylamide quaternisé tel que le produit commercialisé sous la dénomination « GAFQUAT HS 100 » par la société ISP.

(2) les polysaccharides quaternisés décrits plus particulièrement dans les brevets US 3 589 578 et US 4 031 307, tels que les gommes de guar contenant des groupements trialkylammonium cationiques. De tels produits sont commercialisés en particulier sous les dénominations commerciales JAGUAR C13 S, JAGUAR C 15 et JAGUAR C 17 par la société MEYHALL.
(3) les copolymères de vinylpyrrolidone et de vinylimidazole quaternaires ;
(4) les chitosanes ou les sels de ceux-ci ;
(5) les dérivés de cellulose cationiques, tels que les copolymères de cellulose ou de dérivés de cellulose greffés par un monomère hydrosoluble comprenant un ammonium quaternaire et décrits en particulier dans le brevet US 4 131 576, tels que les hydroalkyl celluloses, comme les hydroxyméthyl, hydroxyéthyl ou hydroxypropyl celluloses greffées en particulier par un sel de méthacryloyloxyéthyltriméthylammonium, de méthacrylamidopropyltriméthylammonium ou de diméthyldiallylammonium. Les produits commercialisés correspondant à cette définition sont plus particulièrement les produits commercialisés sous la dénomination « CELQUAT L 200 » et « CELQUAT H 100 » par la National Starch Company.

Parmi les polymères hydrosolubles filmogènes, on peut citer les polymères amphotères suivants :

(1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloroacrylique, et d'un monomère basique dérivé d'un composé vinylique substitué contenant au moins un atome de base tel que plus particulièrement un méthacrylate et acrylate de dialkylaminoalkyle, et un dialkylaminoalkylméthacrylamide et acrylamide. De tels composés sont décrits dans le brevet américain n° 3 836 537.
(2) les polymères comprenant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que les esters, ayant des substituants amine primaire, secondaire, tertiaire et quaternaire, d'acides acrylique et méthacrylique, et le produit de la quaternisation du méthacrylate de diméthylaminoéthyle par du sulfate de diméthyle ou de diéthyle.

(3)les alkoylpolyaminoamides réticulés dérivés totalement ou en partie de polyaminoamides.
(4)les polymères comprenant des motifs zwitterioniques.
(5)le polymère dérivé du chitosane.
(6)les polymères dérivés de la N-carboxyalkylation du chitosane, tels que le N-carboxyméthyl chitosane ou le N-carboxybutyl chitosane commercialisé sous la dénomination « EVALSAN » par la société JAN DEKKER.
(7)les copolymères du ($C_1$-$C_5$)alkylvinyléther/ anhydride maléique partiellement modifié par une semi-amidification par une N,N-dialkylaminoalkylamine, telle que la N,N-diméthylaminopropylamine ou par une semi-estérification par une N,N-dialcanolamine. Ces copolymères peuvent également comporter d'autres comonomères vinyliques tels que le vinylcaprolactame.

Les polymères filmogènes hydrosolubles sont préférablement choisis parmi le groupe constitué par :

- les protéines telles que les protéines d'origine végétale, telles que les protéines de blé ou de soja ; les protéines d'origine animale, telles que la kératine, par exemple les hydrolysats de kératine et les kératines sulfoniques ;
- les polymères anioniques, cationiques, amphotères ou non ioniques de la chitine ou du chitosane ;
- les polymères cellulosiques, tels que l'hydroxyéthyl cellulose, l'hydroxypropyl cellulose, la méthylcellulose, l'éthylhydroxyéthyl cellulose, la carboxyméthyl cellulose, et les dérivés quaternisés de la cellulose ;
- les polymères ou copolymères acryliques tels que les polyacrylates ou les polyméthacrylates ;
- les polymères vinyliques, tels que les polyvinylpyrrolidanes, les copolymères du méthylvinyléther et de l'anhydride maléique, le copolymère de l'acétate de vinyle et de l'acide crotonique, les copolymères de la vinylpyrrolidone et de l'acétate de vinyle ;
- les copolymères de la vinylpyrrolidone et du caprolactame ; les alcools polyvinyliques ;
- les polymères éventuellement modifiés d'origine naturelle, tels que :

- la gomme arabique, la gomme de guar, les dérivés du xanthane, la gomme de karaya ;
- les alginates et les carraghénanes ;
- les glycoaminoglycanes, l'acide hyaluronique et ses dérivés ;
- la gomme laque, la gomme sandaraque, les dammars, l'élémis, les copals ;
- l'acide désoxyribonucléique ;
- les mucopolysaccharides, tels que l'acide hyaluronique, le sulfate de chondroïtine, et des mélanges de ceux-ci.

Ces polymères seront utilisés en particulier si l'on désire une élimination plus ou moins appréciable du film par de l'eau.

Afin d'améliorer la nature filmogène d'un polymère huileux ou aqueux, il est possible d'ajouter au système polymère un agent de coalescence qui sera choisi parmi les agents de coalescence connus.

Selon un mode de réalisation de l'invention, le polymère filmogène peu être choisi parmi les polymères ayant un squelette organique non siliconé greffé par des monomères contenant un polysiloxane. Ces polymères peuvent être liposolubles, lipodispersibles, hydrosolubles ou dispersibles en milieu aqueux, le cas échéant.

Les polymères ayant un squelette organique non siliconé greffé par des monomères contenant un polysiloxane sont constitués d'une chaîne organique principale formée de monomères organiques ne comprenant pas la silicone, sur laquelle on greffe, au sein de ladite chaîne ainsi qu'éventuellement sur au moins l'une des extrémités de celle-ci, au moins un macromère de polysiloxane.

Dans ce qui suit, on doit comprendre que l'expression « macromère de polysiloxane » désigne, ainsi qu'il est généralement accepté, tout monomère contenant une chaîne polymère du type polysiloxane dans sa structure.

Les monomères organiques non siliconés constituant la chaîne principale du polymère siliconé greffé peuvent être choisis parmi les monomères à insaturation éthylénique pouvant être polymérisés par la méthode radicalaire, les monomères polymérisables par polycondensation tels que ceux formant les polyamides, les polyesters, les polyuréthanes, les monomères à cycle ouvrant tels que ceux du type oxazoline ou caprolactone.

Les polymères ayant un squelette organique non siliconé greffé par des monomères contenant un polysiloxane selon la présente invention peuvent être obtenus conformément à toute méthode connue de l'homme de l'art, en particulier par la réaction entre (i) un macromère de polysiloxane de départ correctement fonctionnalisé sur la chaîne de polysiloxane et (ii) un ou plusieurs composés organiques non siliconés, eux même correctement fonctionnalisés par une fonction qui est, capable de réagir avec le groupement ou les groupements fonctionnel(s) porté(s) par ladite silicone en formant une liaison covalente ; un exemple classique d'une telle réaction est la réaction radicalaire entre un groupement vinyle porté à l'une des extrémités de la silicone avec une double liaison d'un monomère à insaturation éthylénique de la chaîne principale.

Les polymères ayant un squelette organique non siliconé greffé par des monomères contenant un polysiloxane selon l'invention sont choisis de préférence parmi ceux décrits dans les brevets US 4 693 935, US 4 728 571 et US 4 972 037 et les demandes de brevet EP-A-0 412 704, EP-A-0 412 707, EP-A-0 640 105 et WO 95/00578. Il concerne des copolymères obtenus par la polymérisation radicalaire à partir de monomères à insaturation éthylénique et de monomères ayant un groupement terminal vinyle, ou bien des copolymères obtenus par la réaction d'une polyoléfine contenant des groupements fonctionnalisés et un macromère de polysiloxane ayant une fonction terminale réactive avec lesdits groupements fonctionnalisés.

Une famille particulière de polymères siliconés greffés convenables pour la mise en oeuvre de la présente invention est constituée par les polymères siliconés greffés contenant :

a) de 0 à 98% en poids d'au moins un monomère lipophile (A) de faible polarité lipophile à insaturation éthylénique, polymérisable par la méthode radicalaire ;
b) de 0 à 98% en poids d'au moins un monomère polaire hydrophile (B) à insaturation éthylénique, copolymérisable avec le monomère ou les monomères du type (A) ;
c) de 0,01 à 50% en poids d'au moins un macromère de polysiloxane (C) de formule générale :

$$X(Y)_nSi(R)_{3-m}Z_m \qquad (I)$$

dans laquelle :

X désigne un groupement vinyle copolymérisable avec les monomères (A) et (B) ;
Y désigne un groupement ayant une liaison divalente ;
R désigne hydrogène, alkyle ou alkoxy en $C_1$-$C_6$, aryle en $C_6$-$C_{12}$;
Z désigne un motif polysiloxane monovalent ayant un poids moléculaire moyen en nombre d'au moins 500 ;
n vaut 0 ou 1 et m est un nombre entier allant de 1 à 3 ; les pourcentages étant calculés par rapport au poids total des monomères (A), (B) et (C).

Ces polymères ont un poids moléculaire moyen en nombre allant de 10 000 à 2 000 000, et préférablement une température de transition vitreuse Tg ou une température de fusion cristalline Tm d'au moins -20°C.

Comme exemples de monomères lipophiles (A), on peut citer les esters d'alcool en $C_1$-$C_{18}$ et de l'acide acrylique ou méthacrylique ; les esters d'alcool en $C_{12}$-$C_{30}$ et de l'acide méthacrylique , le styrène ; les macromères de polystyrène ; l'acétate de vinyle ; le propionate de vinyle ; l'alpha-méthylstyrène ; le tertio-butylstyrène ; le butadiène ; le cyclohexadiène ; le cyclohexadiène [sic] ; l'éthylène ; le propylène ; le vinyltoluène, les esters de l'acide acrylique ou méthacrylique et de 1,1-dihydroperfluoroalcanols ou d'homologues de ceux-ci ; les esters de l'acide acrylique ou méthacrylique et d'omega-hydrofluoroalcanols ; les esters de l'acide acrylique ou méthacrylique et de fluoroalkylsulfonamidoalcools ; les esters de l'acide acrylique ou méthacrylique et de fluoroalkylalcools ; les esters de l'acide acrylique ou méthacrylique et d'alcool-fluoroéthers ; ou des mélanges de ceux-ci. Les monomères (A) préférés sont choisis au sein du groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, l'acrylate de 2-(N-méthylperfluorooctanesulfonamido)éthyle, l'acrylate de 2-(N-butylperfluorooctanesulfonamido)éthyle, ou des mélanges de ceux-ci.

Comme exemples de monomères (B) polaires, on peut citer l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et les hemi-esters de ceux-ci, les (méth) acrylates d'hydroxyalkyle, le chlorure de diallyldiméthylammonium, la vinylpyrrolidone, les éthers vinyliques, les maléimides, la vinylpyridine, le vinylimidazole, les composés polaires vinyliques et hétérocycliques, le sulfonate de styrène, l'alcool allylique, l'alcool vinylique, le vinylcaprolactame ou des mélanges de ceux-ci. Les monomères (B) sont choisis de préférence au sein du groupe constitué par l'acide acrylique, le N,N-diméthylacrylamide, le méthacrylate de diméthylaminoéthyle, le méthacrylate de diméthylaminoéthyle quatemisé, la vinylpyrrolidone, et des mélanges de ceux-ci.

On cite notamment le produit KP 561 ou le KP 562 commercialisé par Shin Etsu tel que le monomère (A) et choisi parmi les esters d'alcool en $C_{18}$-$C_{22}$ et de l'acide méthacrylique.

Les macromères de polysiloxane (C) de formule (I) sont choisis de préférence parmi ceux correspondant à la formule générale (II) suivante :

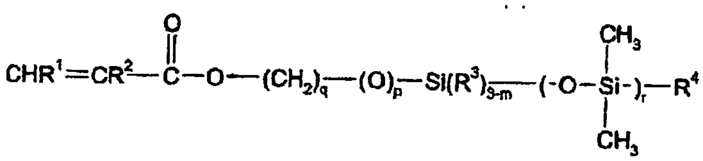 (II)

dans laquelle :

- $R^1$ est hydrogène ou -COOH (préférablement hydrogène) ;
- $R^2$ est hydrogène, méthyle ou -$CH_2COOH$ (préférablement méthyle) ;
- $R^3$ est alkyle, alkoxy ou alkylamino en $C_1$-$C_6$, aryle en $C_6$-$C_{12}$ ou hydroxyle (préférablement méthyle) ;
- $R^4$ est alkyle, alkoxy ou alkylamino en $C_1$-$C_6$, aryle en $C_6$-$C_{12}$ ou hydroxyle (préférablement méthyle) ;
- q est un nombre entier allant de 2 à 6 (préférablement 3) ;
- p vaut 0 ou 1 ;
- r est un nombre entier allant de 5 à 700 ;
- m est un nombre entier allant de 1 à 3 (préférablement 1).

On utilise de préférence les macromères de polysiloxane de formule :

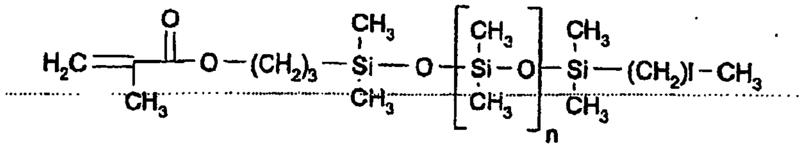

n étant un nombre allant de 5 à 700 et l étant un nombre entier compris entre 0 et 3.

Un mode de réalisation de l'invention consiste en l'utilisation d'un copolymère capable d'être obtenu par une polymérisation radicalaire à partir du mélange de monomères constitué par :

a) 60% en poids d'acrylate de tertio-butyle ;
b) 20% en poids d'acide acrylique ;
c) 20% en poids de macromère siliconé de formule :

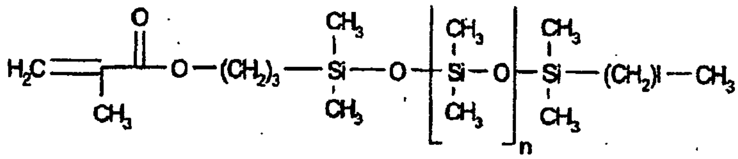

n étant un nombre allant de 5 à 700 et I étant un nombre entier compris entre 0 et 3, les pourcentages en poids étant calculés par rapport au poids total des monomères.

Un autre mode de réalisation particulier de l'invention consiste en l'utilisation d'un copolymère capable d'être obtenu par une polymérisation radicalaire à partir du mélange de monomères constitué par :

a) 80% en poids d'acrylate de tertio-butyle ;
b) 20% en poids de macromère siliconé de formule :

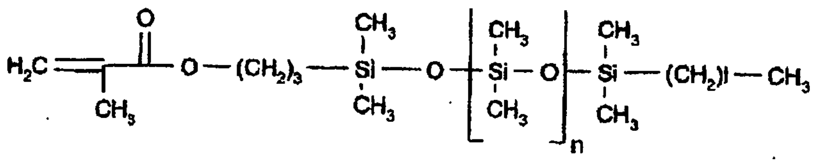

n étant un nombre allant de 5 à 700 et I étant un nombre entier compris entre 0 et 3, les pourcentages en poids étant calculés par rapport au poids total des monomères.

Une autre famille particulière de polymères siliconés greffés ayant un squelette organique non siliconé convenable pour une mise en oeuvre de la présente invention est constituée par les copolymères siliconés greffés capables d'être obtenus par l'extrusion réactive d'un macromère de polysiloxane à fonction terminale réactive sur un polymère du type polyoléfine comprenant des groupements réactifs capables de réagir avec la fonction terminale du macromère de polysiloxane pour former une liaison covalente permettant le greffage de la silicone sur la chaîne principale de la polyoléfine. Ces polymères, ainsi que leur procédé de préparation, sont décrits dans la demande de brevet WO 95/00578.
Les polyoléfines réactives sont choisies de préférence parmi les polyéthylènes ou les polymères de monomères dérivés de l'éthylène, tels que le propylène, le styrène, l'alkylstyrène, le butylène, le butadiène, les (méth)acrylates, les esters vinyliques ou équivalents, comprenant des fonctions réactives capables de réagir avec la fonction terminale du macromère de polysiloxane. Ils sont choisis plus particulièrement parmi les copolymères d'éthylène ou de dérivés d'éthylène et les monomères choisis parmi ceux comprenant une fonction carboxylique tels que l'acide (méth)acrylique ; ceux comprenant une fonction anhydride d'acide tels que l'anhydride de l'acide maléique ; ceux comprenant une fonction chlorure d'acide tels que le chlorure de l'acide (méth)acrylique ; ceux comprenant une fonction ester tels que les esters de l'acide (méth)acrylique ; et ceux comportant une fonction isocyanate.
Les macromères siliconés sont choisis de préférence parmi les polysiloxanes comprenant un groupement fonctionnalisé, à l'extrémité de la chaîne de polysiloxane ou à proximité de l'extrémité de ladite chaîne, choisis au sein du groupe constitué par les alcools, les thiols, les époxy, les amines primaires et secondaires, et tout particulièrement parmi ceux correspondant à la formule générale :

$$T\text{-}(CH_2)_6\text{-}Si\text{-}[\text{-}(OSiR^5R^6)_t\text{-}R^7]_y \qquad (III)$$

dans laquelle T est choisi parmi le groupe constitué par $NH_2$, NHRN, une fonction époxy, OH, SH ; $R^5$, $R^6$, $R^7$ et RN, indépendamment, désignent alkyle en $C_1$-$C_6$, phényle, benzyle ou alkylphényle en $C_6$-$C_{12}$, hydrogène ; s est un nombre allant de 2 à 00 [sic], t est un nombre allant de 0 à 1000 et y est un nombre allant de 1 à 3. Ils ont un poids moléculaire moyen en nombre allant préférablement de 5000 à 300 000, plus préférablement de 8000 à 200 000, et plus particulièrement de 9000 à 40 000.
Selon un mode de réalisation préféré, le polymère filmogène peut être acheté auprès de la Minnesota Mining and Manufacturing Company sous les dénominations commerciales de polymères « Silicone Plus ». Par exemple, le

poly(méthacrylate d'isobutyle-co-FOSEA de méthyle)-g-poly(diméthylsiloxane) est commercialisé sous la dénomination commerciale SA 70-5 IBMMF.

Selon une autre forme préférée de l'invention, le polymère filmogène est choisi parmi les polymères siliconés greffés par des monomères organiques non siliconés. Ces polymères peuvent être liposolubles, lipodispersables, hydrosolubles ou dispersables en milieu aqueux, le cas échéant.

Ledit polymère ou lesdits polymères siliconé(s) greffé(s) ayant un squelette de polysiloxane greffé par des monomères organiques non siliconés contenant une chaîne principale de silicone (ou de polysiloxane $(/SiO-)_n$) sur laquelle se trouve greffé, à l'intérieur de ladite chaîne ainsi qu'éventuellement à l'une au moins de ses extrémités, au moins un groupement organique ne comprenant pas de silicone.

Les polymères ayant un squelette de polysiloxane greffé par des monomères organiques non siliconés selon l'invention peuvent être des produits commerciaux existants ou bien ils peuvent être obtenus par tout moyen connu de l'homme de l'art, en particulier par une réaction entre (i) une silicone de départ correctement fonctionnalisée sur un ou plusieurs de ces atomes de silicium et (ii) un composé organique non siliconé lui-même correctement fonctionnalisé par une fonction qui est capable de réagir avec le groupement ou les groupements fonctionnel(s) porté(s) par ladite silicone en formant une liaison covalente ; un exemple classique d'une telle réaction est la réaction d'hydrosylilation entre des groupements /Si-H et des groupements vinyliques $CH_2=CH-$, voire la réaction entre des groupements thio-fonctionnels -SH avec ces mêmes groupements vinyle.

Des exemples de polymères ayant un squelette de polysiloxane greffé par des monomères organiques non siliconés convenables pour une mise en oeuvre de la présente invention, ainsi que leur méthode spécifique de préparation, sont décrits en particulier dans les demandes de brevet EP-A-0 582 152, WO 93/23009 et WO 95/03776, dont les enseignements sont totalement inclus dans la présente description à titre de références non limitatives.

Selon un mode de réalisation particulièrement préféré de la présente invention, le polymère siliconé, ayant un squelette de polysiloxane greffé par des monomères organiques non siliconés, mis en oeuvre, est constitué du résultat d'une copolymérisation radicalaire entre, d'une part, au moins un monomère organique anionique non siliconé à insaturation éthylénique et/ou un monomère organique hydrophobe non siliconé à insaturation éthylénique et, d'autre part, une silicone présentant dans sa chaîne au moins un groupement fonctionnel, et préférablement plusieurs, capable de réagir avec lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou comme mélanges, parmi les acides carboxyliques insaturés, linéaires ou ramifiés, éventuellement neutralisés partiellement ou totalement sous forme d'un sel, ce ou ces acide(s) carboxylique(s) insaturé(s) pouvant être plus particulièrement l'acide acrylique, l'acide méthacrylique, l'acide maléique, l'acide itaconique, l'acide fumarique et l'acide crotonique. Les sels convenables sont en particulier les sels alcalins, alcalino-terreux et d'ammonium. On notera que, de même, dans le polymère siliconé greffé final, le groupement organique de nature anionique qui est constitué du résultat de l'(homo)polymérisation radicalaire d'au moins un monomère anionique du type acide carboxylique insaturé peut être, après réaction, post-neutralisé par une base (soude, ammoniaque, etc.) pour l'amener sous la forme d'un sel.

Selon la présente invention, les monomères hydrophobes à insaturation éthylénique sont de préférence choisis, seuls ou comme mélange, parmi les esters de l'acide acrylique d'alcanols et/ou les esters de l'acide méthacrylique d'alcanols. Les alcanols sont de préférence en $C_1$-$C_{30}$ et plus particulièrement en $C_1$-$C_{22}$. Les monomères préférés sont choisis parmi le groupe constitué par le (méth)acrylate d'isooctyle, le (méth)acrylate d'isononyle, le (méth)acrylate de 2-éthylhexyle, le (méth)acrylate de lauryle, le (méth)-acrylate d'isopentyle, le (méth)acrylate de n-butyle, le (méth) acrylate d'isobutyle, le (méth)acrylate de méthyle, le (méth)acrylate de tertio-butyle, le (méth)acrylate de tridécyle et le (méth)acrylate de stéaryle, ou des mélanges de ceux-ci.

Une famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule IV ci-dessous :

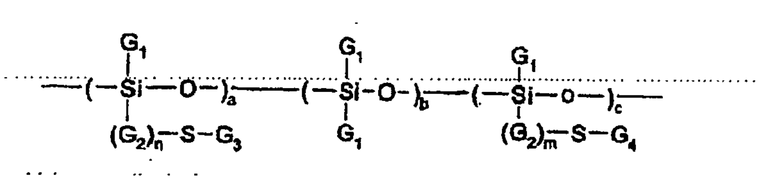

(IV)

dans laquelle les radicaux $G_1$, identiques ou différents, représentent hydrogène ou un radical alkyle en $C_1$-$C_{10}$, voire un radical phényle ; les radicaux $G_2$, identiques ou différents, représentent représente [sic] un groupement alkylène en $C_1$-$C_{10}$; $G_3$ représente un reste polymère résultant de l'(homo)polymérisation d'au moins un monomère anionique à insaturation éthylénique ; $G_4$ représente un reste polymère résultant de l'(homo)polymérisation d'au moins un monomère

d'au moins un monomère hydrophobe [sic] à insaturation éthylénique ; m et n sont égaux à 0 ou 1 ; a est un nombre entier allant de 0 à 50 ; b est un nombre entier pouvant être compris entre 10 et 350, c est un nombre entier allant de 0 à 50 ; à condition que l'un des paramètres a et c soit différent de 0.

Le motif de formule (IV) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène ;
- $G_3$ représente un radical polymère résultant de l'(homo)polymérisation d'au moins un monomère du type acide carboxylique à insaturation éthylénique, préférablement l'acide acrylique et/ou l'acide méthacrylique ;
- $G_4$ représente un radical polymère résultant de l'(homo)polymérisation d'au moins un monomère du type (méth) acrylate d'alkyle en $C_1$-$C_{10}$, préférablement le (méth)acrylate d'isobutyle ou de méthyle.

Des exemples de polymères siliconés correspondant à la formule (IV) sont en particulier des polydiméthylsiloxanes (PDMS) sur lesquels sont greffés, par l'intermédiaire d'une liaison secondaire du type thiopropylène, des motifs polymères mixtes du type acide poly(méth)acrylique et du type poly(méth)acrylate d'alkyle.

D'autres exemples de polymères siliconés correspondant à la formule (IV) sont en particulier des polydiméthylsiloxanes (PDMS) sur lesquels sont greffés, par l'intermédiaire d'une liaison secondaire du type thiopropylène, des motifs polymères du type poly(méth)acrylate d'isobutyle.

De tels polymères comportent les polymères comprenant au moins un groupement de formule :

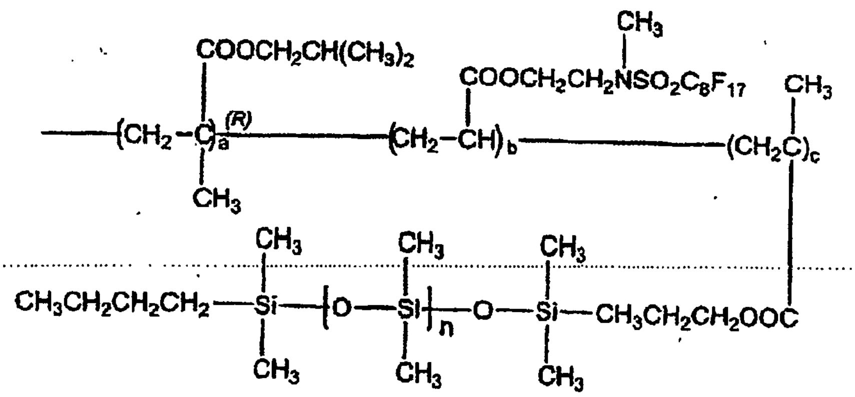

dans laquelle

a, b et c, pouvant être identiques ou différents, sont chacun un nombre allant de 1 à 100 000 ; et les groupements terminaux, pouvant être identiques ou différents, sont choisis chacun parmi les groupements alkyle linéaires en $C_1$-$C_{20}$, les groupements alkyle à chaîne ramifiée en $C_3$-$C_{20}$, les groupements aryle en $C_3$-$C_{20}$, les groupements alkoxy linéaires en $C_1$-$C_{20}$ et les groupements alkoxy ramifiés en $C_3$-$C_{20}$.

De tels polymères sont divulgués dans les brevets US n° 4 972 037, 5 061 481, 5 209 924, 5 849 275 et 6 033 650, et WO 93/23446 et WO 95/06078.

Une autre famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (V) ci-dessous :

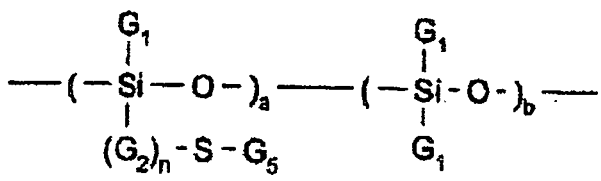

(V)

dans laquelle les radicaux $G_1$ et $G_2$ ont la même signification que ci-dessus; $G_5$ représente un reste polymère résultant de l'(homo)polymérisation d'au moins un monomère d'au moins un monomère hydrophobe [sic] à insaturation éthylénique ou de la copolymérisation d'au moins un monomère anionique à insaturation éthylénique et d'au moins un monomère

hydrophobe à insaturation éthylénique ; n est égal à 0 ou 1 ; a est un nombre entier allant de 0 à 50 ; b est un nombre entier pouvant être compris entre 10 et 350 ; à condition que a soit différent de 0.

Le motif de formule (V) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène.

La masse moléculaire en nombre des polymères siliconés ayant un squelette de polysiloxane greffé par des monomères organiques non siliconés de l'invention varie préférablement d'environ 10 000 à 1 000 000, et encore plus préférablement d'environ 10000 à 100000.

La composition peut contenir de 2 à 60% en poids, mieux de 5 à 60%, préférablement de 2 à 30% en poids de matière sèche de polymère filmogène. Plus généralement, la quantité totale de polymère doit être en quantité suffisante pour former sur la peau et/ou les lèvres un film cohésif capable de suivre les mouvements de la peau et/ou des lèvres sans se décoller ou craquer.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

La composition selon l'invention peut comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol où le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges.
- parmi les tensioactifs anioniques : les acides gras en $C_{16}$-$C_{30}$ neutralisés par les amines, l'ammoniaque ou les sels alcalins, et leurs mélanges.

Selon un mode de réalisation, on utilise de préférence des tensioactifs permettant l'obtention d'émulsion huile-dans-eau ou cire-dans-eau.

La composition selon l'invention comprend un milieu liquide organique cosmétiquement acceptable (tolérance, toxicologie et toucher acceptables).

Selon un mode de réalisation particulièrement préféré, le milieu liquide organique de la composition contient au moins un solvant organique, qui est le ou un des solvants de polymérisation du polymère séquencé tel que décrit précédemment. Avantageusement, ledit solvant organique est le liquide majoritaire en poids dans le milieu liquide organique de la composition cosmétique.

Selon un mode de mise en oeuvre, le milieu solvant organique comprend des corps gras liquides à température ambiante (25°C en général) appelés huiles. Ces corps gras liquides peuvent être d'origine animale, végétale, minérale ou synthétique.

Comme huiles utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène

hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoatès, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

Le milieu liquide organique de la composition selon l'invention peut également comprendre un ou plusieurs solvants organiques, cosmétiquement acceptables (tolérance, toxicologie et toucher acceptables).

Ces solvants peuvent être généralement présents en une teneur allant de 0,1 à 90%, de préférence encore de 10 à 90% en poids, par rapport au poids total de la composition, et mieux de 30 à 90 %.

Comme solvants utilisables dans la composition de l'invention, on peut citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

La composition selon l'invention peut comprendre au moins une cire. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides. Les corps pâteux sont généralement des composés hydrocarbonés comme les lanolines et leurs dérivés ou encore des PDMS.

La nature et la quantité des corps gras solides sont fonction des propriétés mécaniques et des textures recherchées. A titre indicatif, la composition peut contenir de 0 à 50 % en poids de cires, par rapport au poids total de la composition et mieux de 1 à 30 % en poids. Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les

oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de polo-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut se présenter notamment sous forme de stick, de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les sticks de rouge à lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara ou laque pour cheveux).

La composition selon l'invention peut également être un produit de soin de la peau du corps et du visage, notamment un produit solaire ou de coloration de la peau (tel qu'un autobronzant).

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray.

Selon un mode de réalisation, l'invention a pour objet une composition de revêtement des fibres kératiniques (telles que les cils, les sourcils, les cheveux ) comprenant un milieu liquide organique, au moins une phase aqueuse et au moins un polymère éthylénique séquencé linéaire filmogène et une dispersion de particules de polymère filmogène tels que précédemment décrits.

Avantageusement, le polymère filmogène dispersible dans l'eau est choisi parmi les polyuréthanes, les polyuréthane-acryliques, les polyacryliques, les polyesters de (meth)acrylique, les polyvinylpyrrolidone, les polyuréthanes-polyvinylpyrrolidones, les polyester-polyuréthanes, les polyéther-polyuréthanes, les polyurées, les polyurée/polyuréthanes, et leurs mélanges tels que définis plus haut.
Avantageusement, la composition comprend au moins un second agent filmogène choisi parmi les polymère hydrosolubles tels que les dérivés de cellulose cationiques et/ou les polymères éventuellement modifiés d'origine naturelle tel que la gomme arabique.
De préférence, ladite composition comprend une cire et de préférence encore, elle comprend un tensioactif.
Une telle composition peut se présenter sous différentes formes : par exemple, sous la forme d'émulsions diphasiques cire-dans-eau ou eau-dans-cire, de dispersions aqueuses ou anhydres.
Avantageusement, la composition est une composition de revêtement des cils ou mascara.
La présente invention a également pour objet un ensemble cosmétique comprenant :

- un récipient délimitant au moins un compartiment, ledit récipient étant fermé par un élément de fermeture ; et
- une composition telle que décrite précédemment disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.
L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.
Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.
Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.
L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.
Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).
Le récipient peut être à parois rigides ou à parois déformables, notamment sous forme d'un tube ou d'un flacon tube.
Le récipient peut comprendre des moyens destinés à provoquer ou faciliter la distribution de la composition. A titre d'exemple, le récipient peut être à parois déformables .de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.
Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.
Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

Les exemples qui suivent illustrent de manière non limitative les compositions selon l'invention.

**Exemple 4 : Rouge à lèvres liquide**

| INGREDIENTS | % MASSIQUE |
|---|---|
| Polymère de l'exemple 2 | 50,0 |
| Silice (Aerosil R 972®, Degussa) | 5,0 |
| Isododécane gélifié par un copolymère éthylène/propylène/ styrène et un copolymère butylène/ éthylène/styrène (Versagel® MD 970, Penreco) | 7,0 |
| Polyisobutène hydrogéné | 2,1 |
| Octyldodécanol | 0,9 |
| Phényltriméthicone (DC 556, 20 cSt, Dow Corning) | 2,1 |
| Isododécane | 28,3 |
| Copolymère vinylpyrrolidone/1-eicosène (Antaron V-220®, ISP) | 1,2 |
| Pigments | 3,0 |
| Parfum | q.s. |

D'autre part, son application se fait sans difficulté avec un applicateur mousse et conduit à un dépôt homogène de bonne tenue.

**Exemple 5: Composition solaire**

| Ingrédients | (% en poids) |
|---|---|
| Glycérine | 6 |
| Propyleneglycol | 6 |
| Copolymère Acrylates/$C_{10}$-$C_{30}$ alkylacrylate PEMUMEN TR-2 (Noveon) | 0,3 |
| Polymère ammonium polyacryloyldimethyltaurate (HOSTACERIN AMPS -Clariant) | 0,3 |
| Cyclohexasiloxane (DOW CORNING 246 FLUID -Dow Corning) | 6 |
| Gomme de Xanthane RHODICARE XC (Rhodia) | 0,1 |
| Terephtalydene Dicamphor sulfonic Acid (MEXORYL SX - Chimex) | 1,5 |
| Triéthanolamine | q.s. |
| Octocrylene(UVINUL N539 -BASF) | 10 |
| Butylmethoxydibenzoylmethane (Parsol1789 - Roche Vitamines) | 2,5 |
| Drometrizole Trisiloxane (MEXORYL XL - Chimex) | 1,5 |
| $C_{12}$-$C_{15}$ alkyl benzoate (FINSOLV TN - Witco) | 4 |
| Polymère de l'exemple 1 | 1 |

(suite)

| Ingrédients | (% en poids) |
|---|---|
| Triéthanolamine | 0,35 |
| Conservateur et sequestrant | q.s. |
| Eau | q.s.p.100 |
| | |

**Exemple 6 à 11 : Mascaras émulsions**

On a préparé les compositions de mascara suivantes selon l'invention et l'art antérieur : La composition de l'exemple 6 selon l'art antérieur ne comprend pas de polymère séquencé ni de dispersion aqueuse de particules de polymère filmogène.
La composition de l'exemple 10 comprend une dispersion aqueuse de particules de polymère filmogène mais pas de polymère séquencé.
Les compositions des exemples 7 à 9 et 11 selon l'invention comprennent un polymère séquencé et une dispersion aqueuse de particules de polymère filmogène.

| | Exemple 6 (Comparatif) | Exemple 7 (Selon l'invention) | Exemple 8 (Selon l'invention) | Exemple 9 (Selon l'invention) | Exemple 10 (Comparatif) | Exemple 11 (Selon l'invention) |
|---|---|---|---|---|---|---|
| Cire de candellila | 20 | 5 | 5 | 5 | 5 | |
| Dispersion aqueuse de polyuréthane à 38% en MA (« Avalure UR-450® » de la société Goodrich) | | 8,3 (M A*) | | | | |
| Copolymère acrylate d'éthyle/méthacrylate de méthyle (80/20) en dispersion aqueuse à 50 % MA (« Daitosol 5000 AD® » de DAITO) | | | 10 (M A) | | | |
| Copolymère acrylique et styrène/acrylique endispersion aqueuse à 40% en MA (« Syntran 5760 » de la société Interpolymer) | | | | 8,7 (M A) | 17,42 (M A) | 6,37 (M A) |
| Polymère séquencé de l'exemple 3 | | 10 (MA) | 10 (M A) | 10 (M A) | | 15 (MA) |
| Acide stéarique | 5,8 | | | | | |
| Stéarate de triéthanolamine | 2,9 | | | | | |
| Oxyde de fer noir | 8 | | | | | |
| Hydroxyéthylcellulose | 0,9 | | | | | |
| Gomme arabique | 3,4 | | | | | |

(suite)

| | Exemple 6 (Comparatif) | Exemple 7 (Selon l'invention) | Exemple 8 (Selon l'invention) | Exemple 9 (Selon l'invention) | Exemple 10 (Comparatif) | Exemple 11 (Selon l'invention) |
|---|---|---|---|---|---|---|
| Eau, conservateurs | Qsp 100 | | | | | |
| *MA : Matière active | | | | | | |

Pour chaque composition, on a mesuré l'extrait sec, la charge in vitro et la tenue, selon les méthodes décrites ci-après.

La charge in vitro est mesurée par gravimétrie sur des éprouvettes de cheveux caucasiens courbes (30 cheveux longs de 1 cm répartis sur une distance de 1cm). L'éprouvette est maquillée en réalisant 3x10 passages de mascara espacés de 2 minutes avec reprise de produit entre chaque série de 10.
L'éprouvette est séchée 10min à température ambiante puis pesée.
Cette mesure est réalisée sur 6 éprouvettes
La charge est en fait la quantité de matière déposée sur l'éprouvette = masse éprouvette maquillée - masse éprouvette nue.
La charge moyenne est la moyenne des mesures réalisées sur les 6 éprouvettes.

La teneur en matière sèche, c'est à dire la teneur en matière non volatile, ou extrait sec des compositions est mesuré sur une balance Mettler Toledo HG 53 (Halogen Moisture Analyzer). Un échantillon de mascara (2-3g) est déposé sur une coupelle en aluminium et subit une température de 120°C pendant 60 minutes. La mesure de l'extrait sec correspond au suivi de la masse de l'échantillon en fonction du temps .La teneur finale en solides est donc le pourcentage de la masse finale (au bout de 60 min) par rapport à la masse initiale : ES = (masse finale / masse initiale) X 100.

La tenue du film formé par la composition selon l'invention est évaluée par mesure de la résistance à l'eau, en fonction du temps, d'un film de composition étalé sur une plaque de verre et soumis à une agitation en milieu aqueux. Le protocole est le suivant :

> A température ambiante (25°C), On étale une couche de composition de 300 μm d'épaisseur (avant séchage) d'une surface de 9 cm X 9 cm sur une plaque de verre d'une surface de 10 cm X 10 cm, puis on laisse sécher pendant 24 heures à 30 °C et 50 % d'humidité relative. Après séchage, on place la plaque dans un cristallisoir d'un diamètre de 19 cm et d'une contenance de 2 litres rempli d'un litre d'eau posé sur un agitateur magnétique chauffant vendu sous la dénomination RCT basic par la société IKA labor technik. On place ensuite sur le film un barreau aimanté cylindrique lisse en PTFE (longueur 6 cm ; diamètre 1 cm). On règle la vitesse d'agitation en position 5. La température de l'eau est controlée à l'aide d'un thermomètre à la température de 20 °C ou de 40 °C. Au temps $t_0$ = 0, on débute l'agitation. On mesure le temps t (exprimé en minutes) au bout duquel le film commence à se détacher ou se décoller de la plaque ou lorsque l'on observe un trou de la taille du barreau magnétique d'agitation, c'est-à-dire lorsque le trou a un diamètre de 6 cm. La résistance à l'eau du film correspond au temps t mesuré.

On obtient les résultats suivants

| | Exemple 6 (Comparatif) | Exemple 7 (Selon l'invention) | Exemple 8 (Selon l'invention) | Exemple 9 (Selon l'invention) | Exemple 10 (Comparatif) | Exemple 11 (Selon l'invention) |
|---|---|---|---|---|---|---|
| Extrait sec mesuré (%) | 38,8 | 43,8 | 45,2 | 44,9 | 41,6 | 41,8 |
| Charge in vitro (mg) | | 11.90 ± 1.34 | 12.5 ± 1.42 | 10.18 ± 1.54 | 7.43 ± 0.65 | 11.17 ± 0.74 |
| Tenue | 55" | 1'24" | 2'24" | 6'26" | 38' | 19' |

On constate que les compositions selon l'invention des exemples 7 à 9 et 11 comprenant l'association d'une dispersion aqueuse de particules de polymères filmogène et d'un polymère séquencé présentent bonne tenue, meilleure que celle de la composition de l'exemple 10 qui ne comprend pas de polymère séquencé. En outre, les compositions selon l'invention permettent un maquillage épais des cils car elles présentent un extrait sec et des valeurs de charge in

vitro élevées.

**Exemple 12 : Mascara waterproof**

On a préparé le mascara suivant selon l'invention :

| | |
|---|---|
| Cire de Carnauba | 4,7 |
| Cire d'abeille | 8,2 |
| Cire de son de riz | 2,2 |
| Hectorite modifiée (« Bentone 38V® » d'ELEMENTIS | 5,5 |
| Cire de Paraffine | 2,2 |
| Talc | 1 |
| Copolymère acétate de vinyle/stérarate d'allyle (Mexomère PQ de la société CHIMEX) | 6,7 |
| Polymère séquence de l'exemple 1, | 10 |
| Polylaurate de vinyle (Mexomère PP de la société CHIMEX) | 0,7 |
| Sulfopolyester (Eastmann AQ 55S de Eastmann) | 0,1 |
| Conservateurs | 0,2 |
| Carbonate de propylène | 1,8 |
| Eau | 7 |
| Pigments | 5,2 |
| Isododécane | Qsp 100 |

On a mesuré l'extrait sec, la charge in vitro et la tenue, selon les méthode de mesure décrites précédemment dans la description.

Les résultats qui suivent ont été obtenus

| | |
|---|---|
| **Extrait sec mesuré (%)** | 45,4 |
| **Charge in vitro (Mg)** | 8,9 ± 0,9 |
| **Tenue** | Supérieure à un jour (24h) |

Ce mascara présente une bonne tenue tout en ayant un bon effet épaississant (chargeant) des cils.

## Revendications

**1.** Composition cosmétique contenant un milieu liquide organique, au moins un polymère séquencé éthylénique linéaire filmogène non elastomère, et au moins un autre agent filmogène soluble ou dispersible dans ledit milieu liquide organique,
ledit polymère séquencé contenant des première et deuxième séquences reliées entre elles par un segment intermédiaire statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ;
et ledit polymère séquencé ayant un indice de polydispersité I supérieur à 2,8.

**2.** Composition cosmétique contenant un milieu liquide organique, au moins une phase aqueuse, au moins un polymère séquencé éthylénique linéaire filmogène exempt de motif styrène et au moins un autre agent filmogène soluble ou

dispersible dans ladite phase aqueuse,
ledit polymère séquencé contenant des première et deuxième séquences reliées entre elles par un segment intermédiaire statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et

la deuxième séquence étant choisie dans une catégorie a), b) ou c) différente de la première séquence ;
et ledit polymère séquencé ayant un indice de polydispersité I supérieur à 2,8.

3. Composition cosmétique selon la revendication 1 ou 2, **caractérisée en ce que** le polymère séquencé est non élastomère.

4. Composition cosmétique selon l'une des revendications précédentes, **caractérisée en ce que** le polymère séquencé est exempt de motif styrène.

5. Composition cosmétique selon l'une des revendications précédentes, **caractérisée en ce que** le polymère séquencé est un polymère éthylénique issu de monomères éthyléniques aliphatiques comprenant une double liaison carbone carbone et au moins un groupement ester -COO- ou amide -CON-.

6. Composition cosmétique selon l'une des revendications précédentes, **caractérisé en ce que** le polymère n'est pas soluble à une teneur en matière active d'au moins 1% en poids dans l'eau ou dans un mélange d'eau et de monoalcools inférieurs linéaires ou ramifiés ayant de 2 à 5 atomes de carbone, sans modification de pH, à température ambiante (25°C).

7. Composition selon la revendication précédente, **caractérisée en ce que** les première et deuxième séquences sont reliées entre elles par un segment intermédiaire ayant une température de transition vitreuse comprise entre les températures de transition vitreuse des première et deuxième séquences.

8. Composition cosmétique selon l'une des revendications précédentes, **caractérisée en ce que** le polymère séquencé contient des première et deuxième séquences incompatibles dans ledit milieu liquide organique.

9. Composition selon l'une quelconque des revendications précédentes, **caractérisée en ce que** la séquence ayant une Tg supérieure ou égale à 40°C est issue en totalité ou en partie de un ou plusieurs monomères, qui sont tels que l'homopolymère préparé à partir de ces monomères a une température de transition vitreuse supérieure ou égale à 40°C.

10. Composition selon la revendication précédente, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$.
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

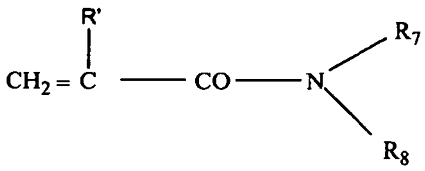

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.

- et leurs mélanges.

**11.** Composition selon la revendication 9 ou 10, **caractérisée en ce que** les monomères dont l'homopolymère correspondant a une température de transition vitreuse supérieure ou égale à 40°C sont choisis parmi le méthacrylate de méthyle, le (méth)acrylate d'isobutyle, le (méth)acrylate d'isobornyle et leurs mélanges.

**12.** Composition selon la revendication précédente, **caractérisée en ce que** la séquence ayant une Tg inférieure ou égale à 20°C est issue en totalité ou en partie de un ou plusieurs monomères, qui sont tel(s) que l'homopolymère préparé à partir de ces monomères a une température de transition vitreuse inférieure ou égale à 20°C.

**13.** Composition selon la revendication précédente, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)-COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5$-CO-O-CH = $CH_2$ où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**14.** Composition selon la revendication 12 ou 13, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**15.** Composition selon l'une des revendications 1 à 8, **caractérisée en ce que** la séquence ayant une Tg comprise entre 20 et 40°C est issue en totalité ou en partie de un ou de plusieurs monomères, qui sont tel(s) que l'homopolymère préparés à partir de ces monomères a une température de transition vitreuse comprise entre 20 et 40°C.

**16.** Composition selon l'une des revendications 1 à 8, **caractérisée en ce que** la séquence ayant une Tg comprise entre 20 et 40°C est issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**17.** Composition selon la revendication 15 ou 16, **caractérisée en ce que** la séquence ayant une Tg comprise entre 20 et 40°C est issue en totalité ou en partie de monomères choisis parmi le méthacrylate de méthyle, l'acrylate et le méthacrylate d'isobornyle, l'acrylate de butyle, l'acrylate d'éthyl-2 hexyle et leurs mélanges.

**18.** Composition selon l'une des revendications 1 à 14, **caractérisée en ce qu'**elle comprend un polymère séquencé comprenant au moins une première séquence et au moins une deuxième séquence, la première séquence ayant une température de transition vitreuse (Tg) supérieure ou égale à 40°C et la deuxième séquence ayant une température de transition vitreuse inférieure ou égale à 20°C.

**19.** Composition selon la revendication précédente, **caractérisée en ce que** la première séquence est issue en totalité ou en partie de un ou plusieurs monomères, qui sont tels que l'homopolymère préparé à partir de ces monomères a une température de transition vitreuse supérieure ou égale à 40°C.

**20.** Composition selon la revendication précédente, **caractérisée en ce que** la première séquence est un copolymère issu de monomères qui sont tels que l'homopolymère préparé à partir de ces monomères a une température de transition vitreuse supérieure ou égale à 40°C.

**21.** Composition selon la revendication 19 ou 20, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

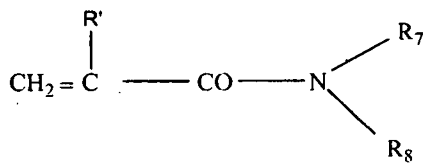

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,
- et leurs mélanges.

**22.** Composition selon l'une des revendications 19 à 21, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**23.** Composition selon l'une des revendications 18 à 22, **caractérisée en ce que** la proportion de la première séquence va de 20 à 90% en poids du polymère, mieux de 30 à 80% et encore mieux de 50 à 70%.

**24.** Composition selon l'une des revendications 18 à 23, **caractérisée en ce que** la deuxième séquence est issue en totalité ou en partie de un ou plusieurs monomères, qui sont tel(s) que l'homopolymère préparé à partir de ces monomères a une température de transition vitreuse inférieure ou égale à 20°C.

**25.** Composition selon l'une des revendications 18 à 24, **caractérisée en ce que** la deuxième séquence est un homopolymère issu de monomères qui sont tel(s) que l'homopolymère préparé à partir de ces monomères a une température de transition vitreuse inférieure ou égale à 20°C.

**26.** Composition selon la revendication 24 ou 25, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;

- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-}CO\text{-}O\text{-}CH = CH_2$ où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**27.** Composition selon l'une des revendications 24 à 26, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**28.** Composition selon l'une des revendications 18 à 27, **caractérisée en ce que** la proportion de la deuxième séquence ayant une Tg inférieure ou égale à 20°C va de 5 à 75% en poids du polymère, mieux de 15 à 50% et encore mieux de 25 à 45%.

**29.** Composition selon l'une des revendications 1 à 17, **caractérisée en ce qu'**elle comprend un polymère séquencé comprenant au moins une première séquence et au moins une deuxième séquence, la première séquence ayant une température de transition vitreuse (Tg) comprise entre 20 et 40°C et la deuxième séquence ayant une température de transition vitreuse inférieure ou égale à 20°C ou une température de transition vitreuse supérieure ou égale à 40°C.

**30.** Composition selon la revendication précédente, **caractérisée en ce que** la première séquence ayant une Tg comprise entre 20 et 40°C est issue en totalité ou en partie de un ou de plusieurs monomères, qui sont tel(s) que l'homopolymère préparé à partir de ces monomères a une température de transition vitreuse comprise entre 20 et 40°C.

**31.** Composition selon la revendication 29 ou 30, **caractérisée en ce que** la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**32.** Composition selon lune des revendications 29 à 31, **caractérisée en ce que** la première séquence ayant une Tg comprise entre 20 et 40°C est issue de monomères choisis parmi le méthacrylate de méthyle, l'acrylate et le méthacrylate d'isobornyle, l'acrylate de butyle, l'acrylate d'éthyl-2 hexyle et leurs mélanges.

**33.** Composition selon l'une des revendications 29 à 32, **caractérisée en ce que** la proportion de la première séquence ayant une Tg comprise entre 20 et 40°C va de 10 à 85% en poids du polymère, mieux de 30 à 80% et encore mieux de 50 à 70%.

**34.** Composition selon l'une quelconque des revendications 29 à 33, **caractérisée en ce que** la deuxième séquence a une Tg supérieure ou égale à 40°C et est issue en totalité ou en partie de un ou plusieurs monomères, qui sont tels que l'homopolymère préparé à partir de ces monomères a une température de transition vitreuse supérieure ou égale à 40°C.

**35.** Composition selon l'une quelconque des revendications 29 à 33, **caractérisée en ce que** la deuxième séquence a une Tg supérieure ou égale à 40°C et est un homopolymère issu de monomères, qui sont tels que l'homopolymère préparé à partir de ces monomères a une température de transition vitreuse supérieure ou égale à 40°C.

**36.** Composition selon l'une des revendications 34 ou 35, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$

dans laquelle $R_2$ représente un groupe cycloalkyl en $C_4$ à $C_{12}$ tel que l'acrylate d'isobornyle ou un groupe tertio butyle,
- les (méth)acrylamides de formule :

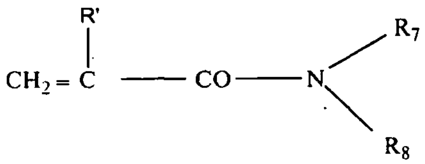

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,
- et leurs mélanges.

**37.** Composition selon l'une des revendications 34 à 36, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**38.** Composition selon l'une des revendications 34 à 37, **caractérisée en ce que** la proportion de la deuxième séquence ayant une Tg supérieure ou égale à 40°C va de 10 à 85%, de préférence de 20 à 70% et mieux de 30 à 70% en poids du polymère.

**39.** Composition selon l'une des revendications 29 à 33, **caractérisée en ce que** la deuxième séquence a une Tg inférieure ou égale à 20°C et est issue en totalité ou en partie de un ou plusieurs monomères, qui sont tel(s) que l'homopolymère préparé à partir de ces monomères a une température de transition vitreuse inférieure ou égale à 20°C.

**40.** Composition selon l'une des revendications 29 à 33, **caractérisée en ce que** la deuxième séquence a une Tg inférieure ou égale à 20°C et est un homopolymère issu de monomères qui sont tel(s) que l'homopolymère préparé à partir de ces monomères a une température de transition vitreuse inférieure ou égale à 20°C.

**41.** Composition selon la revendication 39 ou 40, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertio-butyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$ où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**42.** Composition selon l'une des revendications 39 à 41, **caractérisée en ce que** les monomères dont les homopolymères ont des températures de transition vitreuse inférieures ou égales à 20°C sont choisis parmi les acrylates d'alkyle dont la chaîne alkyle comprend de 1 à 10 atomes de carbone, à l'exception du groupe tertiobutyle.

**43.** Composition selon l'une des revendications 39 à 42, **caractérisée en ce que** la proportion de la séquence ayant une température de transition vitreuse inférieure ou égale à 20°C va de 10 à 85% en poids du polymère, mieux de 20 à 70% et encore mieux de 20 à 50%.

**44.** Composition cosmétique selon l'une des revendications précédentes qui en dépendent, **caractérisée en ce que** la première séquence et/ou la deuxième séquence comprend au moins un monomère additionnel.

**45.** Composition selon la revendication précédente, **caractérisée en ce que** le monomère additionnel est choisi parmi les monomères hydrophiles, les monomères à insaturation éthylénique comprenant un ou plusieurs atomes de silicium et leurs mélanges.

**46.** Composition selon la revendication 44 ou 45, **caractérisée en ce que** le monomère additionnel est choisi parmi :

a) les monomères hydrophiles tels que :

- les monomères à insaturation(s) éthylénique(s) comprenant au moins une fonction acide carboxylique ou sulfonique comme par exemple :

l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine; le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)-COOR_6$
dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,

- les méthacrylates de formule $CH_2 = C(CH_3)-COOR_9$,

$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule $CH_2 = CHCOOR_{10}$,

$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène, et

b) les monomères à insaturation éthylénique comprenant un ou plusieurs atomes de silicium tels que le méthacryloxypropyl triméthoxy silane, le méthacryloxypropyl tris ( triméthylsiloxy ) silane,

- et leurs mélanges.

**47.** Composition selon l'une des revendications 44 ou 45, **caractérisée en ce que** chacune des première et deuxième séquence comprend au moins un monomère additionnel choisi parmi l'acide acrylique, l'acide (méth)acrylique, le méthacrylate de trifluoroéthyle et leurs mélanges.

**48.** Composition selon l'une des revendications 44 ou 45, **caractérisée en ce que** chacune des première et deuxième séquence comprend au moins un monomère choisi parmi les esters d'acide (méth)acrylique et éventuellement au moins un monomère additionnel tel que l'acide (méth)acrylique, et de leurs mélanges.

**49.** Composition selon l'une des revendications 44 ou 45, **caractérisée en ce que** chacune des première et deuxième séquence est issue en totalité d'au moins un monomère choisi parmi les esters d'acide (méth)acrylique et éventuellement d'au moins un monomère additionnel tel que l'acide (méth)acrylique, et de leurs mélanges.

**50.** Composition selon l'une des revendications 44 à 49, **caractérisée en ce que** le ou les monomères additionnel(s) représente(nt) de 1 à 30% en poids du poids total des première et/ou deuxième séquences.

**51.** Composition selon l'une quelconque des revendications précédentes, **caractérisée en ce que** l'écart entre les températures de transition vitreuse (Tg) des première et deuxième séquences est supérieur à 10°C, mieux, supérieur à 20°C, de préférence supérieure à 30°C et mieux supérieure à 40°C.

**52.** Composition selon l'une quelconque des revendications précédentes, **caractérisée en ce que** le polymère séquencé a un indice de polydispersité compris entre 2,8 et 6.

**53.** Composition selon l'une des revendications précédentes, **caractérisée en ce que** le polymère séquencé a une masse moyenne en poids (Mw) est inférieure ou égale à 300 000.

**54.** Composition selon la revendication précédente, **caractérisée en ce que** la masse moyenne en poids (Mw) va de 35 000 à 200 000, et mieux de 45 000 à 150 000.

**55.** Composition selon la revendication précédente, **caractérisée en ce que** la masse moyenne en poids (Mn) est inférieure ou égale à 70 000.

**56.** Composition selon l'une des revendications 53 à 55, dont la masse moyenne en poids (Mn) va de 10 000 à 60 000, et mieux de 12 000 à 50 000.

**57.** Composition selon l'une des revendications précédentes, **caractérisée en ce qu'**elle comprend de 0,1 à 60 % en poids en matière active de polymère séquencé, de préférence de 5 % à 50% en poids, et de préférence encore de 10 à 40 % en poids.

**58.** Composition selon l'une quelconque des revendications 1 et 3 à 57, **caractérisée en ce que** l'agent filmogène est un polymère filmogène soluble dans ledit milieu liquide organique.

**59.** Composition selon la revendication précédente, **caractérisée en ce que** l'agent filmogène est un polymère filmogène liposoluble.

**60.** Composition selon la revendication précédente, **caractérisée en ce que** le polymère filmogène liposoluble est choisi parmi les homopolymères et les copolymères liposolubles et amorphes des oléfines, des cyclooléfines, du butadiène, de l'isoprène, du styrène, des éthers, des esters ou amides vinyliques, des esters ou amides de l'acide (méth)acrylique contenant un groupement alkyle en $C_{4-50}$ linéaire, ramifié ou cyclique, et préférablement amorphes.

**61.** Composition selon la revendication 59, **caractérisée en ce que** le polymère filmogène liposoluble est choisi parmi les homopolymères et les copolymères obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci, en particulier un copolymère d'acrylate d'alkyle/acrylate de cycloalkyle et les copolymères de vinylpyrrolidone/décadécène.

**62.** Composition selon la revendication 59, **caractérisée en ce que** le polymère filmogène liposoluble est choisi parmi les polycondensats amorphes et liposolubles, ne comprenant préférablement pas de groupements donneurs d'interactions hydrogène, en particulier les polyesters ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, solide à température ambiante.

**63.** Composition selon la revendication 59, **caractérisée en ce que** le polymère filmogène liposoluble est choisi parmi les polysaccharides amorphes et liposolubles comprenant des chaînes latérales alkyl(éther ou ester), en particulier l'éthylcellulose, les polymères de silicone-acrylique greffés ayant un squelette siliconé, des greffons acryliques ou ayant un squelette acrylique, les greffons de silicone.

**64.** Composition selon la revendication 59, **caractérisée en ce que** le polymère filmogène liposoluble porte des groupements fluorés.

**65.** Composition selon la revendication 59, **caractérisée en ce que** le polymère filmogène liposoluble est choisi parmi les copolymères de (méth)acrylate d'alkyle/(méth)acrylate de perfluoroalkyle.

**66.** Composition selon la revendication 59, **caractérisée en ce que** le polymère filmogène liposoluble est choisi parmi les polymères ou copolymères résultant de la polymérisation ou la copolymérisation d'un monomère éthylénique, comprenant une ou plusieurs liaisons éthyléniques, de préférence conjuguées.

**67.** Composition selon la revendication précédente, **caractérisée en ce que** le polymère ou copolymère résultant de la polymérisation ou la copolymérisation d'un monomère éthylénique est choisi parmi les polystyrène/copoly(éthylène/ butylène).

**68.** Composition selon la revendication 59, **caractérisée en ce que** le polymère filmogène liposoluble est choisi parmi les polymères ayant un squelette organique non siliconé greffé par des monomères contenant un polysiloxane.

**69.** Composition selon la revendication 59, dans laquelle ledit au moins un polymère filmogène liposoluble est choisi parmi les polymères siliconés greffés par des monomères organiques non siliconés.

**70.** Composition selon l'une quelconque des revendications 1 et 3 à 57, **caractérisée en ce que** l'agent filmogène est un polymère filmogène dispersible dans ledit milieu liquide organique.

**71.** Composition selon la revendication précédente, **caractérisée en ce que** le milieu liquide organique comprend au moins une huile, que l'agent filmogène est dispersible dans ladite huile du, et que l'agent filmogène est sous la forme d'une dispersion non aqueuse de particules de polymère.

**72.** Composition selon l'une quelconque des revendications 2 à 57, **caractérisée en ce que** l'agent filmogène est un polymère filmogène dispersible dans la phase aqueuse.

**73.** Composition selon la revendication précédente, **caractérisée en ce que** le polymère filmogène dispersible dans l'eau est choisi parmi les polyuréthanes, les polyuréthane-acryliques, les polyuréthanes-polyvinylpyrrolidones, les polyester-polyuréthanes, les polyéther-polyuréthanes, les polyurées, les polyurée/polyuréthanes, et leurs mélanges.

**74.** Composition selon la revendication 72, **caractérisée en ce que** le polymère filmogène dispersible dans l'eau est un copolymère de polyuréthane aliphatique, cycloaliphatique ou aromatique, un copolymère de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

**75.** Composition selon la revendication 72, **caractérisée en ce que** le polymère filmogène dispersible dans la phase aqueuse est choisi parmi les polyesters, les polyesteramides, les polyesters à chaîne grasse, les polyamides et les résines d'époxyester.

**76.** Composition selon la revendication 72, **caractérisée en ce que** le polymère filmogène dispersible dans la phase aqueuse est choisi parmi les polymères acryliques, les copolymères acryliques et les polymères vinyliques.

**77.** Composition selon l'une quelconque des revendications précédentes, **caractérisée en ce que** la proportion en agent filmogène va de 2 à 60%, préférablement de 5 à 60%, plus préférablement de 2 à 30% en poids de composé sec par rapport au poids total de la composition.

**78.** Composition cosmétique selon l'une quelconque des revendications précédentes, **caractérisée en ce qu'**elle comprend, en outre, une ou des matières colorantes choisies parmi les colorants hydrosolubles et les matières colorantes pulvérulentes, tels que les pigments, les nacres et les paillettes.

**79.** Composition cosmétique selon l'une quelconque des revendications précédentes, **caractérisée en ce qu'**elle se

présente sous forme de suspension, de dispersion, de solution, de gel, d'émulsion, notamment émulsion huile-dans-eau (H/E) ou eau-dans-huile
(E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre, de stick ou de solide coulé.

**80.** Composition cosmétique selon l'une quelconque des revendications précédentes, **caractérisée en ce qu'**elle se présente sous forme anhydre.

**81.** Composition cosmétique selon l'une quelconque des revendications précédentes, **caractérisée en ce qu'**il s'agit d'une composition de maquillage ou de soin des matières kératiniques.

**82.** Composition selon l'une des revendications précédentes, **caractérisée en ce qu'**il s'agit d'un produit de maquillage des lèvres.

**83.** Composition selon l'une des revendications précédentes, **caractérisée en ce qu'**il s'agit d'un produit de maquillage des yeux.

**84.** Composition selon l'une des revendications précédentes, **caractérisée en ce qu'**il s'agit d'un produit de maquillage du teint.

**85.** Composition selon l'une des revendications précédentes, **caractérisée en ce qu'**il s'agit d'un produit de maquillage des ongles.

**86.** Composition de revêtement des fibres kératiniques, notamment des cils et des sourcils, contenant un milieu liquide organique, au moins une phase aqueuse, au moins un polymère séquencé éthylénique filmogène conforme à l'une des revendications 1 à 56, et au moins un autre agent filmogène soluble ou dispersible dans ladite phase aqueuse,

**87.** Composition selon la revendication précédente, **caractérisée en ce que** l'agent filmogène est un polymère filmogène dispersible dans la phase aqueuse.

**88.** Composition selon la revendication précédente, **caractérisée en ce que** le polymère filmogène dispersible dans l'eau est choisi parmi les polyuréthanes, les polyuréthane-acryliques, les polyuréthanes-polyvinylpyrrolidones, les polyester-polyuréthanes, les polyéther-polyuréthanes, les polyurées, les polyurée/polyuréthanes, et leurs mélanges.

**89.** Composition selon l'une des revendications 86 à 88, **caractérisée en ce qu'**elle comprend une cire.

**90.** Composition selon l'une des revendications 86 à 89, **caractérisée en ce qu'**elle comprend un tensioactif.

**91.** Composition selon l'une des revendications 86 à 90, **caractérisée en ce qu'**elle comprend au moins un second agent filmogène choisi parmi les polymère hydrosolubles.

**92.** Composition selon la revendication précédente, **caractérisée en ce que** le ou les polymères hydrosolubles sont choisis parmi les dérivés de cellulose cationiques et/ou les polymères éventuellement modifiés d'origine naturelle tel que la gomme arabique.

**93.** Composition selon l'une des revendications 86 à 92, **caractérisée en ce qu'**elle comprend un agent de coloration.

**94.** Composition selon l'une des revendications 86 à 93, **caractérisée en ce qu'**elle est un mascara.

**95.** Ensemble cosmétique comprenant :

a) un récipient délimitant au moins un compartiment, ledit récipient étant fermé par un élément de fermeture ; et
b) une composition disposée à l'intérieur dudit compartiment, la composition étant conforme à l'une quelconque des revendications qui précèdent.

**96.** Ensemble cosmétique selon la revendication précédente, **caractérisé en ce que** le récipient est formé, au moins pour partie, en au moins un matériau thermoplastique.

**97.** Ensemble cosmétique selon la revendication 95, **caractérisé en ce que** le récipient est formé, au moins pour partie, en au moins un matériau non thermoplastique, notamment en verre ou en métal.

**98.** Ensemble selon l'une quelconque des revendications 95 à 97, **caractérisé en ce que**, en position fermée du récipient, l'élément de fermeture est vissé sur le récipient.

**99.** Ensemble selon l'une quelconque des revendications 95 à 97, **caractérisé en ce que**, en position fermée du récipient, l'élément de fermeture est couplé au récipient autrement que par vissage, notamment par encliquetage, collage, ou soudage.

**100.** Ensemble selon l'une quelconque des revendications 95 à 99, **caractérisé en ce que** la composition est sensiblement à la pression atmosphérique à l'intérieur du compartiment.

**101.** Ensemble selon l'une quelconque des revendications 95 à 99, **caractérisé en ce que** la composition est pressurisée à l'intérieur du récipient.

**102.** Procédé cosmétique de maquillage ou de soin des matières kératiniques comprenant l'application sur les matières kératiniques d'une composition cosmétique selon l'une des revendications 1 à 94.

## Claims

**1.** Cosmetic composition comprising an organic liquid medium, at least one non-elastomeric film-forming ethylenic linear block polymer, and at least one other film former which is soluble or dispersible in the said organic liquid medium, the said block polymer containing first and second blocks connected to one another by an intermediate random segment comprising at least one constituent monomer of the first block and at least one constituent monomer of the second block,
the first block of the polymer being selected from:

- a) a block with a Tg of greater than or equal to 40°C,
- b) a block with a Tg of less than or equal to 20°C,
- c) a block with a Tg between 20 and 40°C, and

the second block being selected from a category a), b) or c) different from the first block; and
the said block polymer having a polydispersity index I of greater than 2.8.

**2.** Cosmetic composition comprising an organic liquid medium, at least one aqueous phase, at least one film-forming ethylenic linear block polymer free from styrene units, and at least one other film former which is soluble or dispersible in the said aqueous phase,
the said block polymer containing first and second blocks connected to one another by an intermediate random segment comprising at least one constituent monomer of the first block and at least one constituent monomer of the second block,
the first block of the polymer being selected from:

- a) a block with a Tg of greater than or equal to 40°C,
- b) a block with a Tg of less than or equal to 20°C,
- c) a block with a Tg between 20 and 40°C, and

the second block being selected from a category a), b) or c) different from the first block; and
the said block polymer having a polydispersity index I of greater than 2.8.

**3.** Cosmetic composition according to Claim 1 or 2, **characterized in that** the block polymer is non-elastomeric.

**4.** Cosmetic composition according to one of the preceding claims, **characterized in that** the block polymer is free from styrene units.

**5.** Cosmetic composition according to one of the preceding claims, **characterized in that** the block polymer is an ethylenic polymer obtained from aliphatic ethylenic monomers comprising a carbon-carbon double bond and at least one ester group -COO- or amide group -CON-.

6. Cosmetic composition according to one of the preceding claims, **characterized in that** the polymer is not soluble at an amount of active substance of at least 1% by weight in water or in a mixture of water and linear or branched lower monoalcohols having 2 to 5 carbon atoms, without a change in pH, at ambient temperature (25°C).

7. Composition according to the preceding claim, **characterized in that** the first and second blocks are connected to one another by an intermediate segment having a glass transition temperature between the glass transition temperatures of the first and second blocks.

8. Cosmetic composition according to one of the preceding claims, **characterized in that** the block polymer comprises first and second blocks which are incompatible in the said organic liquid medium.

9. Composition according to any one of the preceding claims, **characterized in that** the block with a Tg of greater than or equal to 40°C is obtained totally or partly from one or more monomers which are such that the homopolymer prepared from these monomers has a glass transition temperature of greater than or equal to 40°C.

10. Composition according to the preceding claim, **characterized in that** the monomers whose corresponding homopolymer has a glass transition temperature of greater than or equal to 40°C are selected from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group, or $R_1$ represents a $C_4$ to $C_{12}$ cycloalkyl group;
- acrylates of formula $CH_2 = CH\text{-}COOR_2$ in which $R_2$ represents a $C_4$ to $C_{12}$ cycloalkyl group, such as isobornyl acrylate or a tert-butyl group;
- (meth)acrylamides of formula:

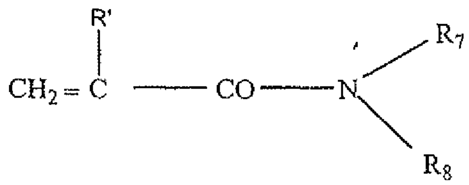

where $R_7$ and $R_8$, which are identical or different, each represent a hydrogen atom or a linear or branched alkyl group having from 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group and R' denotes H or methyl;
- and mixtures thereof.

11. Composition according to Claim 9 or 10, **characterized in that** the monomers whose corresponding homopolymer has a glass transition temperature of greater than or equal to 40°C are selected from methyl methacrylate, isobutyl (meth)acrylate, isobornyl (meth)acrylate, and mixtures thereof.

12. Composition according to the preceding claim, **characterized in that** the block with a Tg of less than or equal to 20°C is obtained totally or partly from one or more monomers which are such that the homopolymer prepared from these monomers has a glass transition temperature of less than or equal to 20°C.

13. Composition according to the preceeding claim, **characterized in that** the monomers whose corresponding homopolymer has a glass transition temperature of less than or equal to 20°C are selected from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms selected from O, N and S is (are) optionally intercalated;
- methacrylates of formula $CH_2 = C(CH_3)\text{-}COOR_4$, $R_4$ representing a linear or branched $C_6$ to $C_{12}$ unsubstituted alkyl group, in which one or more heteroatoms selected from O, N and S is (are) optionally intercalated;
- vinyl esters of formula $R_5\text{-}CO\text{-}O\text{-}CH = CH_2$ where $R_5$ represents a linear or branched $C_4$ to $C_{12}$ alkyl group;
- $C_4$ to $C_{12}$ alkyl vinyl ethers;
- N-($C_4$ to $C_{12}$ alkyl) acrylamides, such as N-octylacrylamide;

- and mixtures thereof.

**14.** Composition according to Claim 12 or 13, **characterized in that** the monomers whose corresponding homopolymer has a glass transition temperature of less than or equal to 20°C are selected from alkyl acrylates in which the alkyl chain contains from 1 to 10 carbon atoms, with the exception of the tert-butyl group.

**15.** Composition according to one of Claims 1 to 8, **characterized in that** the block with a Tg of between 20 and 40°C is obtained totally or partly from one or more monomers which are such that the homopolymer prepared from these monomers has a glass transition temperature of between 20 and 40°C.

**16.** Composition according to one of Claims 1 to 8, **characterized in that** the block with a Tg of between 20 and 40°C is obtained totally or partly from monomers which are such that the corresponding homopolymer has a Tg of greater than or equal to 40°C and from monomers which are such that the corresponding homopolymer has a Tg of less than or equal to 20°C.

**17.** Composition according to Claim 15 or 16, **characterized in that** the block with a Tg of between 20 and 40°C is obtained totally or partly from monomers selected from methyl methacrylate, isobornyl acrylate and methacrylate, butyl acrylate, 2-ethylhexyl acrylate, and mixtures thereof.

**18.** Composition according to one of Claims 1 to 14, **characterized in that** it comprises a block polymer comprising at least one first block and at least one second block, the first block having a glass transition temperature (Tg) of greater than or equal to 40°C and the second block having a glass transition temperature of less than or equal to 20°C.

**19.** Composition according to the preceding claim, **characterized in that** the first block is obtained totally or partly from one or more monomers which are such that the homopolymer prepared from these monomers has a glass transition temperature of greater than or equal to 40°C.

**20.** Composition according to the preceding claim, **characterized in that** the first block is a copolymer obtained from monomers which are such that the homopolymer prepared from these monomers has a glass transition temperature of greater than or equal to 40°C.

**21.** Composition according to Claim 19 or 20, **characterized in that** the monomers whose corresponding homopolymer has a glass transition temperature of greater than or equal to 40°C are selected from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group, or $R_1$ represents a $C_4$ to $C_{12}$ cycloalkyl group;
- acrylates of formula $CH_2 = CH\text{-}COOR_2$ in which $R_2$ represents a $C_4$ to $C_{12}$ cycloalkyl group, such as isobornyl acrylate or a tert-butyl group;
- (meth)acrylamides of formula:

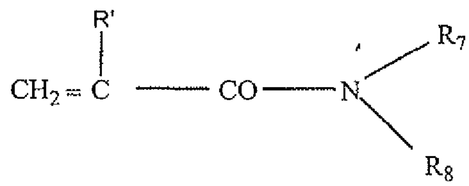

where $R_7$ and $R_8$, which are identical or different, each represent a hydrogen atom or a linear or branched alkyl group having from 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group and R' denotes H or methyl;
- and mixtures thereof.

**22.** Composition according to one of Claims 19 to 21, **characterized in that** the monomers whose corresponding homopolymer has a glass transition temperature of greater than or equal to 40°C are selected from methyl methacrylate, isobutyl methacrylate, isobornyl (meth)acrylate, and mixtures thereof.

**23.** Composition according to one of Claims 18 to 22, **characterized in that** the proportion of the first block ranges from 20% to 90%, more preferably from 30% to 80% and better still from 50% to 70% by weight of the polymer.

**24.** Composition according to one of Claims 18 to 23, **characterized in that** the second block is obtained totally or partly from one or more monomers which are such that the homopolymer prepared from these monomers has a glass transition temperature of less than or equal to 20°C.

**25.** Composition according to one of Claims 18 to 24, **characterized in that** the second block is a homopolymer obtained from monomers which are such that the homopolymer prepared from these monomers has a glass transition temperature of less than or equal to 20°C.

**26.** Composition according to Claim 24 or 25, **characterized in that** the monomers whose corresponding homopolymer has a glass transition temperature of less than or equal to 20°C are selected from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms selected from O, N and S is (are) optionally intercalated;
- methacrylates of formula $CH_2 = C(CH_3)\text{-}COOR_4$, $R_4$ representing a linear or branched $C_6$ to $C_{12}$ unsubstituted alkyl group, in which one or more heteroatoms selected from O, N and S is (are) optionally intercalated;
- vinyl esters of formula $R_5\text{-}CO\text{-}O\text{-}CH = CH_2$ where $R_5$ represents a linear or branched $C_4$ to $C_{12}$ alkyl group;
- $C_4$ to $C_{12}$ alkyl vinyl ethers;
- N-($C_4$ to $C_{12}$ alkyl) acrylamides, such as N-octylacrylamide;
- and mixtures thereof.

**27.** Composition according to one of Claims 24 to 26, **characterized in that** the monomers whose corresponding homopolymer has a glass transition temperature of less than or equal to 20°C are selected from alkyl acrylates in which the alkyl chain contains from 1 to 10 carbon atoms, with the exception of the tert-butyl group.

**28.** Composition according to one of Claims 18 to 27, **characterized in that** the proportion of the second block with a Tg of less than or equal to 20°C ranges from 5% to 75% by weight of the polymer, better still from 15% to 50% and even better still from 25% to 45%.

**29.** Composition according to one of Claims 1 to 17, **characterized in that** it comprises a block polymer comprising at least one first block and at least one second block, the first block having a glass transition temperature (Tg) of between 20 and 40°C and the second block having a glass transition temperature of less than or equal to 20°C or a glass transition temperature of greater than or equal to 40°C.

**30.** Composition according to the preceding claim, **characterized in that** the first block with a Tg of between 20 and 40°C is obtained totally or partly from one or more monomers which are such that the homopolymer prepared from these monomers has a glass transition temperature of between 20 and 40°C.

**31.** Composition according to Claim 29 or 30, **characterized in that** the first block with a Tg of between 20 and 40°C is a copolymer obtained from monomers which are such that the corresponding homopolymer has a Tg of greater than or equal to 40°C and from monomers which are such that the corresponding homopolymer has a Tg of less than or equal to 20°C.

**32.** Composition according to one of Claims 29 to 31, **characterized in that** the first block with a Tg of between 20 and 40°C is obtained from monomers selected from methyl methacrylate, isobornyl acrylate and methacrylate, butyl acrylate, 2-ethylhexyl acrylate, and mixtures thereof.

**33.** Composition according to one of Claims 29 to 32, **characterized in that** the proportion of the first block with a Tg of between 20 and 40°C ranges from 10% to 85%, better still from 30% to 80% and even better still from 50% to 70% by weight of the polymer.

**34.** Composition according to any one of Claims 29 to 33, **characterized in that** the second block has a Tg of greater than or equal to 40°C and is obtained totally or partly from one or more monomers which are such that the homopolymer prepared from these monomers has a glass transition temperature of greater than or equal to 40°C.

**35.** Composition according to any one of Claims 29 to 33, **characterized in that** the second block has a Tg of greater than or equal to 40°C and is a homopolymer obtained from monomers which are such that the homopolymer prepared from these monomers has a glass transition temperature of greater than or equal to 40°C.

**36.** Composition according to either of Claims 34 and 35, **characterized in that** the monomers whose corresponding homopolymer has a glass transition temperature of greater than or equal to 40°C are selected from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group, or $R_1$ represents a $C_4$ to $C_{12}$ cycloalkyl group;
- acrylates of formula $CH_2 = CH\text{-}COOR_2$ in which $R_2$ represents a $C_4$ to $C_{12}$ cycloalkyl group, such as isobornyl acrylate or a tert-butyl group;
- (meth)acrylamides of formula:

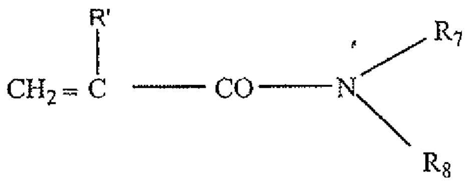

where $R_7$ and $R_8$, which are identical or different, each represent a hydrogen atom or a linear or branched alkyl group having from 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group and R' denotes H or methyl;
- and mixtures thereof.

**37.** Composition according to one of Claims 34 to 36, **characterized in that** the monomers whose corresponding homopolymer has a glass transition temperature of greater than or equal to 40°C are selected from methyl methacrylate, isobutyl methacrylate, isobornyl (meth)acrylate, and mixtures thereof.

**38.** Composition according to one of Claims 34 to 37, **characterized in that** the proportion of the second block with a Tg of greater than or equal to 40°C ranges from 10% to 85%, preferably from 20% to 70% and better still from 30% to 70% by weight of the polymer.

**39.** Composition according to one of Claims 29 to 33, **characterized in that** the second block has a Tg of less than or equal to 20°C and is obtained totally or partly from one or more monomers which are such that the homopolymer prepared from these monomers has a glass transition temperature of less than or equal to 20°C.

**40.** Composition according to one of Claims 29 to 33, **characterized in that** the second block has a Tg of less than or equal to 20°C and is a homopolymer obtained from monomers which are such that the homopolymer prepared from these monomers has a glass transition temperature of less than or equal to 20°C.

**41.** Composition according to Claim 39 or 40, **characterized in that** the monomers whose corresponding homopolymer has a glass transition temperature of less than or equal to 20°C are selected from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms selected from O, N and S is (are) optionally intercalated;
- methacrylates of formula $CH_2 = C(CH_3)\text{-}COOR_4$, $R_4$ representing a linear or branched $C_6$ to $C_{12}$ unsubstituted alkyl group, in which one or more heteroatoms selected from O, N and S is (are) optionally intercalated;
- vinyl esters of formula $R_5\text{-}CO\text{-}O\text{-}CH = CH_2$ where $R_5$ represents a linear or branched $C_4$ to $C_{12}$ alkyl group;
- $C_4$ to $C_{12}$ alkyl vinyl ethers;
- N-($C_4$ to $C_{12}$ alkyl) acrylamides, such as N-octylacrylamide;
- and mixtures thereof.

**42.** Composition according to one of Claims 39 to 41, **characterized in that** the monomers whose homopolymers have

glass transition temperatures of less than or equal to 20°C are selected from alkyl acrylates in which the alkyl chain contains from 1 to 10 carbon atoms, with the exception of the tert-butyl group.

**43.** Composition according to one of Claims 39 to 42, **characterized in that** the proportion of the block with a glass transition temperature of less than or equal to 20°C ranges from 10% to 85% by weight of the polymer, better still from 20% to 70% and even better still from 20% to 50%.

**44.** Cosmetic composition according to one of the preceding claims, **characterized in that** the first block and/or the second block comprises at least one additional monomer.

**45.** Composition according to the preceding claim, **characterized in that** the additional monomer is selected from hydrophilic monomers and ethylenically unsaturated monomers comprising one or more silicon atoms, and mixtures thereof.

**46.** Composition according to Claim 44 or 45, **characterized in that** the additional monomer is selected from:

a) hydrophilic monomers such as:

- ethylenically unsaturated monomers comprising at least one carboxylic or sulphonic acid function, for instance:

acrylic acid, methacrylic acid, crotonic acid, maleic anhydride, itaconic acid, fumaric acid, maleic acid, acrylamidopropanesulphonic acid, vinylbenzoic acid, vinylphosphoric acid, and salts thereof;

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof;
- methacrylates of formula $CH_2 = C(CH_3)\text{-}COOR_6$ in which $R_6$ represents a linear or branched alkyl group containing from 1 to 4 carbon atoms, such as a methyl, ethyl, propyl or isobutyl group, the said alkyl group being substituted by one or more substituents selected from hydroxyl groups (for instance 2-hydroxypropyl methacrylate and 2-hydroxyethyl methacrylate) and halogen atoms (Cl, Br, I or F), such as trifluoroethyl methacrylate;
- methacrylates of formula $CH_2 = C(CH_3)\text{-}COOR_9$, $R_9$ representing a linear or branched $C_6$ to $C_{12}$ alkyl group in which one or more heteroatoms selected from O, N and S is (are) optionally intercalated, the said alkyl group being substituted by one or more substituents selected from hydroxyl groups and halogen atoms (Cl, Br, I or F);
- acrylates of formula $CH_2 = CHCOOR_{10}$,
$R_{10}$ representing a linear or branched $C_1$ to $C_{12}$ alkyl group substituted by one or more substituents selected from hydroxyl groups and halogen atoms (Cl, Br, I or F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit from 5 to 30 times, for example methoxy-POE, or
$R_{10}$ represents a polyoxyethylenated group comprising from 5 to 30 ethylene oxide units; and

b) ethylenically unsaturated monomers comprising one or more silicon atoms, such as methacryloxypropyltrimethoxysilane and methacryloxypropyltris(trimethylsiloxy)silane;

- and mixtures thereof.

**47.** Composition according to either of Claims 44 and 45, **characterized in that** each of the first and second blocks comprises at least one additional monomer selected from acrylic acid, (meth)acrylic acid, trifluoroethyl methacrylate, and mixtures thereof.

**48.** Composition according to either of Claims 44 and 45, **characterized in that** each of the first and second blocks comprises at least one monomer selected from esters of (meth)acrylic acid and optionally at least one additional monomer such as (meth)acrylic acid, and mixtures thereof.

**49.** Composition according to either of Claims 44 and 45, **characterized in that** each of the first and second blocks is obtained totally from at least one monomer selected from esters of (meth)acrylic acid and optionally at least one

additional monomer such as (meth)acrylic acid, and mixtures thereof.

50. Composition according to one of Claims 44 to 49, **characterized in that** the additional monomer or monomers represent(s) from 1% to 30% by weight of the total weight of the first and/or second blocks.

51. Composition according to any one of the preceding claims, **characterized in that** the difference between the glass transition temperatures (Tg) of the first and second blocks is greater than 10°C, better still greater than 20°C, very preferably greater than 30°C and better still greater than 40°C.

52. Composition according to any one of the preceding claims, **characterized in that** the block polymer has a polydispersity index of between 2.8 and 6.

53. Composition according to one of the preceding claims, **characterized in that** the block polymer has a weight-average mass (Mw) of less than or equal to 300 000.

54. Composition according to the preceding claim, **characterized in that** the weight-average mass (Mw) ranges from 35 000 to 200 000 and better still from 45 000 to 150 000.

55. Composition according to the preceding claim, **characterized in that** the number-average mass (Mn) is less than or equal to 70 000.

56. Composition according to one of Claims 53 to 55, whose number-average mass (Mn) ranges from 10 000 to 60 000 and better still from 12 000 to 50 000.

57. Composition according to one of the preceding claims, **characterized in that** it contains from 0.1% to 60% by weight of block polymer active substance, preferably from 5% to 50% by weight, and more preferably from 10% to 40% by weight.

58. Composition according to any one of Claims 1 and 3 to 57, **characterized in that** the film former is a film-forming polymer which is soluble in the said organic liquid medium.

59. Composition according to the preceding claim, **characterized in that** the film former is a fat-soluble film-forming polymer.

60. Composition according to the preceding claim, **characterized in that** the fat-soluble film-forming polymer is selected from the fat-soluble, amorphous homopolymers and copolymers of olefins, of cycloolefins, of butadiene, of isoprene, of styrene, of vinyl ethers, esters or amides, or of (meth)acrylic acid esters or amides comprising a linear, branched or cyclic $C_{4-50}$ alkyl group, which are preferably amorphous.

61. Composition according to Claim 59, **characterized in that** the fat-soluble film-forming polymer is selected from homopolymers and copolymers obtained from monomers selected from the group consisting of isooctyl (meth) acrylate, isononyl (meth)acrylate, 2-ethylhexyl (meth) acrylate, lauryl (meth)acrylate, isopentyl (meth)acrylate, n-butyl (meth)acrylate, isobutyl (meth)acrylate, methyl (meth) acrylate, tert-butyl (meth)acrylate, tridecyl (meth)acrylate, stearyl (meth) acrylate, or mixtures thereof, especially an alkyl acrylate/cycloalkyl acrylate copolymer and vinylpyrrolidone/decadecene copolymers.

62. Composition according to Claim 59, **characterized in that** the fat-soluble film-forming polymer is selected from amorphous and fat-soluble polycondensates, preferably not comprising any groups donating hydrogen interactions, in particular polyesters having $C_{4-50}$ alkyl side chains or else polyesters resulting from the condensation of fatty acid dimers, or even polyesters comprising a silicone-based segment in the form of a block, graft or end group, which is solid at ambient temperature.

63. Composition according to Claim 59, **characterized in that** the fat-soluble film-forming polymer is selected from amorphous and fat-soluble polysaccharides comprising alkyl (ether or ester) side chains, in particular ethylcellulose, or silicone-acrylic graft polymers having a silicone skeleton and acrylic grafts or having an acrylic skeleton and silicone grafts.

64. Composition according to Claim 59, **characterized in that** the fat-soluble film-forming polymer bears fluoro groups.

**65.** Composition according to Claim 59, **characterized in that** the fat-soluble film-forming polymer is selected from alkyl (meth)acrylate/perfluoroalkyl (meth)acrylate copolymers.

**66.** Composition according to Claim 59, **characterized in that** the fat-soluble film-forming polymer is selected from polymers or copolymers resulting from the polymerization or copolymerization of an ethylenic monomer comprising one or more ethylenic, preferably conjugated, bonds.

**67.** Composition according to the preceding claim, **characterized in that** the polymer or copolymer resulting from the polymerization or copolymerization of an ethylenic monomer is selected from polystyrene/copoly(ethylene/butylene) s.

**68.** Composition according to Claim 59, **characterized in that** the fat-soluble film-forming polymer is selected from polymers containing a non-silicone organic skeleton grafted with monomers containing a polysiloxane.

**69.** Composition according to Claim 59, in which the said at least one fat-soluble film-forming polymer is selected from silicone polymers grafted with non-silicone organic monomers.

**70.** Composition according to any one of Claims 1 and 3 to 57, **characterized in that** the film former is a film-forming polymer which is dispersible in the said organic liquid medium.

**71.** Composition according to the preceding claim, **characterized in that** the organic liquid medium comprises at least one oil, **in that** the film former is dispersible in the said oil and **in that** the film former is in the form of a non-aqueous dispersion of polymer particles.

**72.** Composition according to any one of Claims 2 to 57, **characterized in that** the film former is a film-forming polymer which is dispersible in the aqueous phase.

**73.** Composition according to the preceding claim, **characterized in that** the water-dispersible film-forming polymer is selected from polyurethanes, polyurethane-acrylics, polyurethane-polyvinylpyrrolidones, polyester-polyurethanes, polyether-polyurethanes, polyureas, polyurea/polyurethanes, and mixtures thereof.

**74.** Composition according to Claim 72, **characterized in that** the water-dispersible film-forming polymer is an aliphatic, cycloaliphatic or aromatic polyurethane copolymer, or a polyurea/polyurethane or polyurea copolymer comprising, alone or as a mixture:

- at least one block of linear or branched aliphatic and/or cycloaliphatic and/or aromatic polyester origin, and/or
- at least one block of aliphatic and/or cycloaliphatic and/or aromatic polyether origin, and/or
- at least one substituted or unsubstituted, branched or unbranched silicone block, for example polydimethylsiloxane or polymethylphenylsiloxane, and/or
- at least one block comprising fluoro groups.

**75.** Composition according to Claim 72, **characterized in that** the film-forming polymer which is dispersible in the aqueous phase is selected from polyesters, polyesteramides, fatty-chain polyesters, polyamides and epoxy ester resins.

**76.** Composition according to Claim 72, **characterized in that** the film-forming polymer dispersible in the aqueous phase is selected from acrylic polymers, acrylic copolymers and vinyl polymers.

**77.** Composition according to any one of the preceding claims, **characterized in that** the proportion of film former ranges from 2% to 60%, preferably from 5% to 60%, more preferably from 2% to 30% by weight of dry compound relative to the total weight of the composition.

**78.** Cosmetic composition according to any one of the preceding claims, **characterized in that** it further comprises one or more colorants selected from water-soluble dyes and pulverulent colorants such as pigments, nacres and flakes.

**79.** Cosmetic composition according to any one of the preceding claims, **characterized in that** it is in the form of a suspension, dispersion, solution, gel, emulsion, especially oil-in-water (O/W) or water-in-oil (W/O), or multiple (W/O/W or polyol/O/W or O/W/O), emulsion, or in the form of a cream, paste or mousse, or a vesicle dispersion,

particularly of ionic or nonionic lipids, or a two-phase or multi-phase lotion, a spray, powder or paste, especially a flexible paste or anhydrous paste, or a stick or cast solid.

**80.** Cosmetic composition according to any one of the preceding claims, **characterized in that** it is in anhydrous form.

**81.** Cosmetic composition according to any one of the preceding claims, **characterized in that** it is a composition for making up or caring for keratin materials.

**82.** Composition according to one of the preceding claims, **characterized in that** it is a lip makeup product.

**83.** Composition according to one of the preceding claims, **characterized in that** it is an eye makeup product.

**84.** Composition according to one of the preceding claims, **characterized in that** it is a complexion makeup product.

**85.** Composition according to one of the preceding claims, **characterized in that** it is a nail makeup product.

**86.** Coating composition for keratin fibres, particularly the eyelashes and eyebrows, comprising an organic liquid medium, at least one aqueous phase, at least one film-forming ethylenic block polymer in accordance with one of Claims 1 to 56 and at least one other film former soluble or dispersible in the said aqueous phase.

**87.** Composition according to the preceding claim, **characterized in that** the film former is a film-forming polymer dispersible in the aqueous phase.

**88.** Composition according to the preceding claim, **characterized in that** the water-dispersible film-forming polymer is selected from polyurethanes, polyurethane-acrylics, polyurethane-polyvinylpyrrolidones, polyester-polyurethanes, polyether-polyurethanes, polyureas, polyurea/polyurethanes, and mixtures thereof.

**89.** Composition according to one of Claims 86 to 88, **characterized in that** it comprises a wax.

**90.** Composition according to one of Claims 86 to 89, **characterized in that** it comprises a surfactant.

**91.** Composition according to one of Claims 86 to 90, **characterized in that** it comprises at least one second film former selected from water-soluble polymers.

**92.** Composition according to the preceding claim, **characterized in that** the water-soluble polymer or polymers is or are selected from cationic cellulose derivatives and/or optionally modified polymers of natural origin such as gum arabic.

**93.** Composition according to one of Claims 86 to 92, **characterized in that** it comprises a colorant.

**94.** Composition according to one of Claims 86 to 93, **characterized in that** it is a mascara.

**95.** Cosmetic kit comprising:

a) a container delimiting at least one compartment, the said container being closed by a closing element; and
b) a composition disposed inside the said compartment, the composition being in accordance with any one of the preceding claims.

**96.** Cosmetic kit according to the preceding claim, **characterized in that** the container is formed, at least partly, of at least one thermoplastic material.

**97.** Cosmetic kit according to Claim 95, **characterized in that** the container is formed, at least partly, of at least one non-thermoplastic material, particularly of glass or of metal.

**98.** Kit according to any one of Claims 95 to 97, **characterized in that**, in the closed position of the container, the closing element is screwed onto the container.

**99.** Kit according to any one of Claims 95 to 97, **characterized in that**, in the closed position of the container, the

closing element is coupled to the container other than by screwing, in particular by snap fastening, adhesive bonding or welding.

**100.** Kit according to any one of Claims 95 to 99, **characterized in that** the composition is substantially at the atmospheric pressure inside the compartment.

**101.** Kit according to any one of Claims 95 to 99, **characterized in that** the composition is pressurized inside the container.

**102.** Cosmetic method of making up or caring for keratin materials, comprising the application to the keratin materials of a cosmetic composition according to one of Claims 1 to 94.

**Patentansprüche**

**1.** Kosmetische Zusammensetzung, die ein flüssiges organisches Medium, mindestens ein nicht elastomeres, filmbildendes, lineares, ethylenisches Sequenzpolymer und mindestens einen weiteren, in dem flüssigen organischen Medium löslichen oder dispergierbaren Filmbildner enthält,
wobei das Sequenzpolymer eine erste Sequenz und eine zweite Sequenz enthält, die über ein statistisches Zwischensegment aneinandergebunden sind, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist;
wobei das Sequenzpolymer einen Polydispersitätsindex I größer 2,8 aufweist.

**2.** Kosmetische Zusammensetzung, die ein flüssiges organisches Medium, mindestens eine wässrige Phase, mindestens ein filmbildendes, lineares, ethylenisches Sequenzpolymer ohne Styroleinheit und mindestens einen weiteren, in der wässrigen Phase löslichen oder dispergierbaren Filmbildner enthält,
wobei das Sequenzpolymer eine erste Sequenz und eine zweite Sequenz enthält, die über ein statistisches Zwischensegment aneinandergebunden sind, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist;
wobei das Sequenzpolymer einen Polydispersitätsindex I größer 2,8 aufweist.

**3.** Kosmetische Zusammensetzung nach Anspruch 1 oder 2,
**dadurch gekennzeichnet, dass** das Sequenzpolymer nicht elastomer ist.

**4.** Kosmetische Zusammensetzung nach einem der vorhergehenden Ansprüche, **dadurch gekennzeichnet, dass** das Sequenzpolymer keine Styroleinheit aufweist.

**5.** Kosmetische Zusammensetzung nach einem der vorhergehenden Ansprüche, **dadurch gekennzeichnet, dass** das Sequenzpolymer ein ethylenisches Polymer ist, das von aliphatischen ethylenischen Monomeren abgeleitet ist, die eine Kohlenstoff-Kohlenstoff-Doppelbindung und mindestens eine Estergruppe - COO- oder Amidgruppe -CON- aufweisen.

**6.** Kosmetische Zusammensetzung nach einem der vorhergehenden Ansprüche, **dadurch gekennzeichnet, dass** das Polymer bei einem Gehalt der wirksamen Substanz von mindestens 1 Gew.-% in Wasser oder einem Gemisch von Wasser und linearen oder verzweigten, niederen Monoalkoholen mit 2 bis 5 Kohlenstoffatomen ohne pH-Wert-Änderung bei Raumtemperatur (25 °C) nicht löslich ist.

**7.** Zusammensetzung nach dem vorhergehenden Anspruch,
**dadurch gekennzeichnet, dass** die erste Sequenz und die zweite Sequenz über eine Zwischensequenz aneinander gebunden sind, die eine Glasübergangstemperatur aufweist, die zwischen den Glasübergangstemperaturen der ersten und der zweiten Sequenz liegt.

**8.** Kosmetische Zusammensetzung nach einem der vorhergehenden Ansprüche, **dadurch gekennzeichnet, dass** das Sequenzpolymer eine erste Sequenz und eine zweite Sequenz aufweist, die in dem flüssigen organischen Medium nicht miteinander kompatibel sind.

**9.** Zusammensetzung nach einem der vorhergehenden Ansprüche, **dadurch gekennzeichnet, dass** die Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

**10.** Zusammensetzung nach dem vorhergehenden Anspruch,
**dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

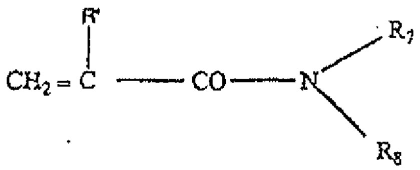

worin $R_7$ und $R_8$, die gleich oder verschieden sein können, jeweils ein Wasserstoffatom oder eine lineare oder verzweigte Alkylgruppe mit 1 bis 12 Kohlenstoffatomen, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
- und deren Gemischen.

**11.** Zusammensetzung nach Anspruch 9 oder 10, **dadurch gekennzeichnet, dass** die Monomere, deren entsprechendes Homopolymer eine Glasübergangstemperatur von größer oder gleich 40 °C aufweist, unter Methylmethacrylat, Isobutyl(meth)acrylat und Isobornyl(meth)acrylat und deren Gemischen ausgewählt sind.

**12.** Zusammensetzung nach dem vorhergehenden Anspruch,
**dadurch gekennzeichnet, dass** die Sequenz mit einer Tg von kleiner oder gleich 20 °C ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**13.** Zusammensetzung nach dem vorhergehenden Anspruch,
**dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),

- Methacrylaten der Formel $CH_2=C(CH_3)-COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5-CO-O-CH=CH_2$,
worin $R_5$ eine lineare oder verzweigte Alkylgruppe mit 4 bis 12 Kohlenstoffatomen bedeutet,
- Alkyl($C_{4-12}$)vinylethern,
- N-Alkyl($C_{4-12}$)acrylamiden, wie N-Octylacrylamid,
- und deren Gemischen.

**14.** Zusammensetzung nach Anspruch 12 oder 13, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, wobei die tert-Butylgruppe ausgenommen ist.

**15.** Zusammensetzung nach einem der Ansprüche 1 bis 8, **dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**16.** Zusammensetzung nach einem der Ansprüche 1 bis 8, **dadurch gekennzeichnet, dass** die Sequenz mit einer Tg zwischen 20 und 40°C ganz oder teilweise von Monomeren, die so sind, dass das aus diesen Monomeren hergestellte Homopolymer eine Glasübergangstemperatur von größer oder gleich 40 °C aufweist, und von Monomeren abgeleitet ist, die so sind, dass das aus diesen Monomeren hergestellte Homopolymer eine Glasübergangstemperatur von kleiner oder gleich 20 °C aufweist.

**17.** Zusammensetzung nach Anspruch 15 oder 16, **dadurch gekennzeichnet, dass** die Sequenz mit einer Tg zwischen 20 und 40°C ganz oder teilweise von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**18.** Zusammensetzung nach einem der Ansprüche 1 bis 14, **dadurch gekennzeichnet, dass** sie ein Sequenzpolymer enthält, das zumindest eine erste Sequenz und zumindest eine zweite Sequenz umfasst, wobei die erste Sequenz eine Glasübergangstemperatur (Tg) von größer oder gleich 40 °C und die zweiten Sequenz eine Glasübergangstemperatur von kleiner oder gleich 20 °C aufweist.

**19.** Zusammensetzung nach dem vorhergehenden Anspruch,
**dadurch gekennzeichnet, dass** die erste Sequenz ganz oder teilweise von einem oder mehreren Monomeren stammt, die so sind, dass das aus diesen Monomeren hergestellte Homopolymer eine Glasübergangstemperatur von größer oder gleich 40 °C aufweist.

**20.** Zusammensetzung nach dem vorhergehenden Anspruch,
**dadurch gekennzeichnet, dass** die erste Sequenz ein Copolymer ist, das von Monomeren abgeleitet ist, die so sind, dass das aus diesen Monomeren hergestellte Homopolymer eine Glasübergangstemperatur von größer oder gleich 40 °C aufweist.

**21.** Zusammensetzung nach Anspruch 19 oder 20, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

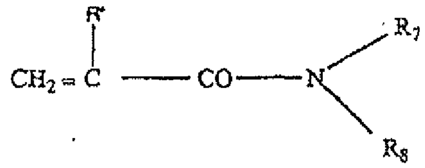

worin $R_7$ und $R_8$, die gleich oder verschieden sein können, jeweils ein Wasserstoffatom oder eine lineare oder verzweigte Alkylgruppe mit 1 bis 12 Kohlenstoffatomen, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
- und deren Gemischen.

**22.** Zusammensetzung nach einem der Ansprüche 19 bis 21,
**dadurch gekennzeichnet, dass** die Monomere, deren entsprechendes Homopolymer eine Glasübergangstemperatur von größer oder gleich 40 °C aufweist, unter Methylmethacrylat, Isobutylmethacrylat und Isobornyl(meth) acrylat und deren Gemischen ausgewählt sind.

**23.** Zusammensetzung nach einem der Ansprüche 18 bis 22,
**dadurch gekennzeichnet, dass** der Anteil der ersten Sequenz im Bereich von 20 bis 90 Gew.-% des Polymers, besser im Bereich von 30 bis 80 Gew.-% und noch besser im Bereich von 50 bis 70 Gew.-% liegt.

**24.** Zusammensetzung nach einem der Ansprüche 18 bis 23,
**dadurch gekennzeichnet, dass** die zweite Sequenz ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**25.** Zusammensetzung nach einem der Ansprüche 18 bis 24,
**dadurch gekennzeichnet, dass** die zweite Sequenz ein Homopolymer ist, das von Monomeren abgeleitet ist, die so sind, dass das aus diesen Monomeren hergestellte Homopolymer eine Glasübergangstemperatur von kleiner oder gleich 20 °C aufweist.

**26.** Zusammensetzung nach Anspruch 24 oder 25, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2=C(CH_3)-COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5-CO-O-CH=CH_2$,
worin $R_5$ eine lineare oder verzweigte Alkylgruppe mit 4 bis 12 Kohlenstoffatomen bedeutet,
- Alkyl($C_{4-12}$)vinylethern,
- N-Alkyl($C_{4-12}$)acrylamiden, wie N-Octylacrylamid,
- und deren Gemischen.

**27.** Zusammensetzung nach einem der Ansprüche 24 bis 26, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, mit Ausnahme der tert-Butylgruppe.

**28.** Zusammensetzung nach einem der Ansprüche 18 bis 27,
**dadurch gekennzeichnet, dass** der Anteil der zweiten Sequenz mit einer Tg von kleiner oder gleich 20 °C im Bereich von 5 bis 75 Gew.-% des Polymers, besser im Bereich von 15 bis 50 Gew.-% und noch besser im Bereich

von 25 bis 45 Gew.-% liegt.

**29.** Zusammensetzung nach einem der Ansprüche 1 bis 17, **dadurch gekennzeichnet, dass** sie ein Sequenzpolymer enthält, das zumindest eine erste Sequenz und zumindest eine zweite Sequenz umfasst, wobei die erste Sequenz eine Glasübergangstemperatur (Tg) zwischen 20 °C und 40 °C aufweist und die zweite Sequenz eine Glasübergangstemperatur von kleiner oder gleich 20 °C oder einer Glasübergangstemperatur von größer oder gleich 40 °C aufweist.

**30.** Zusammensetzung nach dem vorhergehenden Anspruch,
**dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**31.** Zusammensetzung nach Anspruch 29 oder 30, **dadurch gekennzeichnet, dass** die erste Sequenz mit einer Tg zwischen 20 und 40°C ein Copolymer ist, das von Monomeren, die so sind, dass das entsprechende Homopolymer eine Tg von größer oder gleich 40 °C aufweist, und Monomeren abgeleitet ist, die so sind, dass das entsprechende Homopolymer eine Tg von kleiner oder gleich 20 °C aufweist.

**32.** Zusammensetzung nach einem der Ansprüche 29 bis 31,
**dadurch gekennzeichnet, dass** die erste Sequenz mit einer Tg zwischen 20 und 40°C von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**33.** Zusammensetzung einem der Ansprüche 29 bis 32, **dadurch gekennzeichnet, dass** der Anteil der ersten Sequenz mit einer Tg zwischen 20 und 40 °C im Bereich von 10 bis 85 Gew.-% des Polymers, besser im Bereich von 30 bis 80 Gew.-% und noch besser im Bereich von 50 bis 70 Gew.-% liegt.

**34.** Zusammensetzung nach einem der Ansprüche 29 bis 33,
**dadurch gekennzeichnet, dass** die zweite Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

**35.** Zusammensetzung nach einem der Ansprüche 29 bis 33,
**dadurch gekennzeichnet, dass** die zweite Sequenz eine Tg von größer oder gleich 40 °C aufweist und ein Homopolymer ist, das von Monomeren abgeleitet ist, die so sind, dass das aus diesen Monomeren hergestellte Homopolymer eine Glasübergangstemperatur von größer oder gleich 40 °C aufweist.

**36.** Zusammensetzung nach einem der Ansprüche 34 oder 35, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

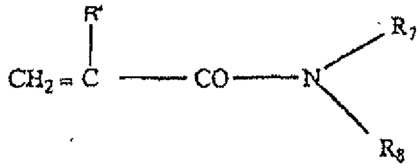

worin $R_7$ und $R_8$, die gleich oder verschieden sein können, jeweils ein Wasserstoffatom oder eine lineare oder verzweigte Alkylgruppe mit 1 bis 12 Kohlenstoffatomen, wie n-Butyl, tert-Butyl, Isopropyl, Isohexyl, Isooctyl oder

Isononyl, bedeuten; oder $R_7$ H bedeutet und $R_8$ eine 1,1-Dimethyl-3-oxobutylgruppe ist, und R' H oder Methyl bedeutet;
- und deren Gemischen.

**37.** Zusammensetzung nach einem der Ansprüche 34 bis 36,
**dadurch gekennzeichnet, dass** die Monomere, deren entsprechendes Homopolymer eine Glasübergangstemperatur von größer oder gleich 40 °C aufweist, unter Methylmethacrylat, Isobutylmethacrylat und Isobornyl(meth)acrylat und deren Gemischen ausgewählt sind.

**38.** Zusammensetzung nach einem der Ansprüche 34 bis 37,
**dadurch gekennzeichnet, dass** der Anteil der zweiten Sequenz mit einer Tg von größer oder gleich 40 °C im Bereich von 10 bis 85 Gew.-%, vorzugsweise im Bereich von 20 bis 70 Gew.-% und besser im Bereich von 30 bis 70 Gew.-% des Polymers liegt.

**39.** Zusammensetzung nach einem der Ansprüche 29 bis 33,
**dadurch gekennzeichnet, dass** die zweite Sequenz eine Tg von kleiner oder gleich 20 °C aufweist und ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**40.** Zusammensetzung nach einem der Ansprüche 29 bis 33,
**dadurch gekennzeichnet, dass** die zweite Sequenz eine Tg von kleiner oder gleich 20 °C aufweist und ein Homopolymer ist, das von Monomeren abgeleitet ist, die so sind, dass das aus diesen Monomeren hergestellte Homopolymer eine Glasübergangstemperatur von kleiner oder gleich 20 °C aufweist.

**41.** Zusammensetzung nach Anspruch 39 oder 40, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2=C(CH_3)-COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH=$CH_2$,
worin $R_5$ eine lineare oder verzweigte Alkylgruppe mit 4 bis 12 Kohlenstoffatomen bedeutet,
- Alkyl($C_{4-12}$)vinylethern,
- N-Alkyl($C_{4-12}$)acrylamiden, wie N-Octylacrylamid,
- und deren Gemischen.

**42.** Zusammensetzung nach einem der Ansprüche 39 bis 41,
**dadurch gekennzeichnet, dass** die Monomere, deren Homopolymere Glasübergangstemperaturen von kleiner oder gleich 20 °C aufweisen, mit Ausnahme der tert-Butylgruppe unter den Alkylacrylaten ausgewählt sind, deren Alkylgruppe 1 bis 10 Kohlenstoffatome aufweist.

**43.** Zusammensetzung nach einem der Ansprüche 39 bis 42,
**dadurch gekennzeichnet, dass** der Anteil der Sequenz mit einer Glasübergangstemperatur von kleiner oder gleich 20 °C im Bereich von 10 bis 85 Gew.-% des Polymers, besser im Bereich von 20 bis 70 Gew.-% und noch besser im Bereich von 20 bis 50 Gew.-% liegt.

**44.** Kosmetische Zusammensetzung nach einem der vorhergehenden Ansprüche, **dadurch gekennzeichnet, dass** die erste Sequenz und/oder die zweite Sequenz mindestens ein ergänzendes Monomer umfassen.

**45.** Zusammensetzung nach dem vorhergehenden Anspruch,
**dadurch gekennzeichnet, dass** das ergänzende Monomer unter den hydrophilen Monomeren, Monomeren mit ethylenisch ungesättigter Bindung, die ein oder mehrere Siliciumatome enthalten, und deren Gemischen ausgewählt ist.

**46.** Zusammensetzung nach Anspruch 44 oder 45, **dadurch gekennzeichnet, dass** das ergänzende Monomer ausgewählt ist unter:

a) hydrophilen Monomeren, wie:

- Monomeren mit einer oder mehreren ethylenisch ungesättigten Bindungen, die mindestens eine Carbonsäurefunktion oder Sulfonsäurefunktion aufweisen, wie beispielsweise:

Acrylsäure, Methacrylsäure, Crotonsäure, Maleinsäureanhydrid, Itaconsäure, Fumarsäure, Maleinsäure, Acrylamidopropansulfonsäure, Vinylbenzoesäure, Vinylphosphorsäure, und deren Salzen,

- Monomeren mit einer oder mehreren ethylenisch ungesättigten Bindungen, die mindestens eine tertiäre Aminofunktion aufweisen, wie beispielsweise 2-Vinylpyridin, 4-Vinylpyridin, Dimethylaminoethylmethacrylat, Diethylaminoethylmethacrylat und Dimethylaminopropylmethacrylamid, und deren Salzen,
- Methacrylaten der Formel $CH_2=C(CH_3)-COOR_6$,
worin $R_6$ eine lineare oder verzweigte Alkylgruppe mit 1 bis 4 Kohlenstoffatomen bedeutet, wie Methyl, Ethyl, Propyl oder Isobutyl, wobei die Alkylgruppe mit einem oder mehreren Substituenten substituiert ist, die unter den Hydroxygruppen (z.B. 2-Hydroxypropylmethacrylat und 2-Hydroxyethylmethacrylat) und Halogenatomen (Cl, Br, I, F), wie Trifluorethylmethacrylat, ausgewählt sind,
- Methacrylaten der Formel $CH_2=C(CH_3)-COOR_9$,
worin $R_9$ eine lineare oder verzweigte Alkylgruppe mit 6 bis 12 Kohlenstoffatomen bedeutet, in die optional ein oder mehrere Heteroatome, die unter O, N und S ausgewählt sind, eingebaut ist (sind), wobei die Alkylgruppe mit einem oder mehreren Substituenten substituiert ist, die unter den Hydroxygruppen und Halogenatomen (Cl, Br, I, F) ausgewählt sind,
- Acrylaten der Formel $CH_2=CHCOOR_{10}$,
worin $R_{10}$ eine lineare oder verzweigte Alkylgruppe mit 1 bis 12 Kohlenstoffatomen bedeutet, die mit einem oder mehreren Substituenten substituiert ist, die unter den Hydroxygruppen und den Halogenatomen (Cl, Br, I und F) ausgewählt sind, wie 2-Hydroxypropylacrylat und 2-Hydroxyethylacrylat, oder $R_{10}$ eine Gruppe $C_{1-12}$-Alkyl-O-POE (Polyoxyethylen) bedeutet, wobei die Oxyethyleneinheit 5 bis 30 Mal wiederholt wird, beispielsweise Methoxy-POE, oder $R_{10}$ eine polyethoxylierte Gruppe bedeutet, die 5 bis 30 Ethylenoxideinheiten aufweist, und

b) ethylenisch ungesättigten Monomeren, die ein oder mehrere Siliciumatome enthalten, wie Methacryloxypropyltrimethoxysilan und Methacryloxypropyltris(trimethylsiloxy)silan,
und deren Gemischen.

**47.** Zusammensetzung nach einem der Ansprüche 44 oder 45, **dadurch gekennzeichnet, dass** die erste und die zweite Sequenz jede mindestens ein zusätzliches Monomer enthält, das unter Acrylsäure, (Meth)acrylsäure und Trifluorethylmethacrylat und deren Gemischen ausgewählt ist.

**48.** Zusammensetzung nach einem der Ansprüche 44 oder 45, **dadurch gekennzeichnet, dass** die erste und die zweite Sequenz jede mindestens ein zusätzliches Monomer enthält, das unter (Meth)acrylsäureestern ausgewählt ist, und optional mindestens ein ergänzendes Monomer, wie (Meth)acrylsäure, und deren Gemische.

**49.** Zusammensetzung nach einem der Ansprüche 44 oder 45,
**dadurch gekennzeichnet, dass** die erste und die zweite Sequenz jede vollständig von mindestens einem Monomer, das unter (Meth)acrylsäureestern ausgewählt ist, und optional mindestens einem ergänzenden Monomer, wie (Meth) acrylsäure, und deren Gemischen abgeleitet ist.

**50.** Zusammensetzung nach einem der Ansprüche 44 bis 49,
**dadurch gekennzeichnet, dass** das oder die zusätzliche(n) Monomer(e) 1 bis 30 Gew.-% des Gesamtgewichts der ersten und/oder zweiten Sequenz ausmachen.

**51.** Zusammensetzung nach einem der vorhergehenden Ansprüche, **dadurch gekennzeichnet, dass** die Differenz der Glasübergangstemperaturen (Tg) der ersten und der zweiten Sequenz größer als 10 °C, besser größer als 20 °C, vorzugsweise größer als 30 °C und besonders bevorzugt größer als 40 °C ist.

**52.** Zusammensetzung nach einem der vorhergehenden Ansprüche, **dadurch gekennzeichnet, dass** das Sequenzpolymer einen Polydispersitätsindex im Bereich von 2,8 bis 6 aufweist.

**53.** Zusammensetzung nach einem der vorhergehenden Ansprüche, **dadurch gekennzeichnet, dass** das Sequenzpolymer eine gewichtsmittlere Molmasse (Mw) von kleiner oder gleich 300 000 aufweist.

**54.** Zusammensetzung nach dem vorhergehenden Anspruch,
**dadurch gekennzeichnet, dass** die gewichtsmittlere Molmasse (Mw) im Bereich von 35 000 bis 200 000 und noch besser im Bereich von 45 000 bis 150 000 liegt.

**55.** Zusammensetzung nach dem vorhergehenden Anspruch,
**dadurch gekennzeichnet, dass** die gewichtsmittlere Molmasse (Mn) kleiner oder gleich 70 000 ist.

**56.** Zusammensetzung nach einem der Ansprüche 53 bis 55, deren gewichtsmittlere Molmasse (Mn) im Bereich von 10 000 bis 60 000 und noch besser im Bereich von 12 000 bis 50 000 liegt.

**57.** Zusammensetzung nach einem der vorhergehenden Ansprüche, **dadurch gekennzeichnet, dass** sie 0,1 bis 60 Gew.-% wirksame Substanz des Sequenzpolymers, vorzugsweise 5 bis 50 Gew.-% und noch bevorzugter 10 bis 40 Gew.-% enthält.

**58.** Zusammensetzung nach einem der Ansprüche 1 und 3 bis 57, **dadurch gekennzeichnet, dass** der Filmbildner ein in dem flüssigen organischen Medium lösliches filmbildendes Polymer ist.

**59.** Zusammensetzung nach dem vorhergehenden Anspruch,
**dadurch gekennzeichnet, dass** der Filmbildner ein fettlösliches filmbildendes Polymer ist.

**60.** Zusammensetzung nach dem vorhergehenden Anspruch,
**dadurch gekennzeichnet, dass** das fettlösliche filmbildende Polymer ausgewählt ist unter den fettlöslichen und amorphen Homopolymeren und Copolymeren von Olefinen, Cycloolefinen, Butadien, Isopren, Styrol, Vinylethern, Vinylestern oder Vinylamiden, Estern oder Amiden von (Meth)acrylsäure, die eine lineare, verzweigte oder cyclische $C_{4-50}$-Alkylgruppe enthalten, wobei sie vorzugsweise amorph sind.

**61.** Zusammensetzung nach Anspruch 59, **dadurch gekennzeichnet, dass** das fettlösliche filmbildende Polymer unter den Homopolymeren und Copolymeren ausgewählt ist, die ausgehend von Monomeren erhalten werden, die unter Isooctyl(meth)acrylat, Isononyl(meth)acrylat, 2-Ethylhexyl(meth)acrylat, Lauryl(meth)acrylat, Isopentyl(meth)acrylat, n-Butyl(meth)acrylat, Isobutyl(meth)acrylat, Methyl(meth)acrylat, tert-Butyl(meth)acrylat, Tridecyl(meth)acrylat, Stearyl(meth)acrylat oder deren Gemischen ausgewählt sind, und insbesondere einem Alkylacrylat/Cycloalkylacrylat-Copolymer und den Vinylpyrrolidon/Decadecen-Copolymeren.

**62.** Zusammensetzung nach Anspruch 59, **dadurch gekennzeichnet, dass** das fettlösliche filmbildende Polymer unter den fettlöslichen und amorphen Polykondensaten ausgewählt ist, die vorzugsweise keine mit Wasserstoff wechselwirkende Gruppen enthalten, insbesondere Polyestern mit $C_{4-50}$-Alkyl-Seitengruppen oder Polyestern, die durch Kondensation von Fettsäuredimeren gebildet werden, oder auch Polyestern, die ein bei Raumtemperatur festes Siliconsegment in der Form eines Segments, eines Pfropfzweigs oder einer endständigen Gruppe enthalten.

**63.** Zusammensetzung nach Anspruch 59, **dadurch gekennzeichnet, dass** das fettlösliche filmbildende Polymer unter den fettlöslichen und amorphen Polysacchariden ausgewählt ist, die seitliche Alkyl(ester oder ether)gruppen enthalten, insbesondere Ethylcellulose, gepfropften Silicon-Acrylpolymeren mit Silicongrundgerüst und Acrylpfropfzweigen oder Acrylgrundgerüst und Siliconpfropfzweigen.

**64.** Zusammensetzung nach Anspruch 59, **dadurch gekennzeichnet, dass** das fettlösliche filmbildende Polymer fluorierte Gruppen trägt.

**65.** Zusammensetzung nach Anspruch 59, **dadurch gekennzeichnet, dass** das fettlösliche filmbildende Polymer unter den Alkyl(meth)acrylat/Perfluoralkyl(meth)acrylat-Copolymeren ausgewählt ist.

**66.** Zusammensetzung nach Anspruch 59, **dadurch gekennzeichnet, dass** das fettlösliche filmbildende Polymer unter den Homopolymeren oder Copolymeren ausgewählt ist, die bei der Polymerisation oder Copolymerisation eines ethylenischen Monomers gebildet werden, das eine oder mehrere ethylenische Bindungen aufweist, die vorzugsweise konjugiert sind.

**67.** Zusammensetzung nach dem vorhergehenden Anspruch,
**dadurch gekennzeichnet, dass** das Homopolymer oder Copolymer, das bei der Polymerisation oder Copolymerisation eines ethylenischen Monomers gebildet wird, unter den Polystyrol/Copoly(ethylen/butylenen) ausgewählt ist.

**68.** Zusammensetzung nach Anspruch 59, **dadurch gekennzeichnet, dass** das fettlösliche filmbildende Polymer unter den Polymeren ausgewählt ist, die ein nicht siliconiertes organisches Grundgerüst aufweisen, das mit ein Polysiloxan enthaltenden Monomeren gepfropft ist.

**69.** Zusammensetzung nach Anspruch 59, **dadurch gekennzeichnet, dass** das fettlösliche filmbildende Polymer unter den Siliconpolymeren ausgewählt ist, die mit organischen, nicht siliconierten Monomeren gepfropft sind.

**70.** Zusammensetzung nach einem der Ansprüche 1 und 3 bis 57,
**dadurch gekennzeichnet, dass** der Filmbildner ein in dem flüssigen organischen Medium dispergierbares filmbildendes Polymer ist.

**71.** Zusammensetzung nach dem vorhergehenden Anspruch,
**dadurch gekennzeichnet, dass** das flüssige organische Medium mindestens ein Öl enthält, **dadurch**, dass der Filmbildner in dem Öl dispergierbar ist, und **dadurch**, dass der Filmbildner in der Form einer nicht wässrigen Dispersion von Polymerpartikel vorliegt.

**72.** Zusammensetzung nach einem der Ansprüche 2 bis 57, **dadurch gekennzeichnet, dass** der Filmbildner ein in der wässrigen Phase dispergierbares filmbildendes Polymer ist.

**73.** Zusammensetzung nach dem vorhergehenden Anspruch,
**dadurch gekennzeichnet, dass** das in Wasser dispergierbare filmbildende Polymer unter den Polyurethanen, Polyurethan-acrylverbindungen, Polyurethanen-polyvinylpyrrolidonen, Polyester-polyurethanen, Polyether-polyurethanen, Polyharnstoffen, Polyharnstoff/Polyurethanen und deren Gemischen ausgewählt ist.

**74.** Zusammensetzung nach Anspruch 72, **dadurch gekennzeichnet, dass** das in Wasser dispergierbare filmbildende Polymer ein aliphatisches, cycloaliphatisches oder aromatisches Polyurethan-Copolymer, ein Polyharnstoff/ Polyurethan-Copolymer oder Polyharnstoff-Copolymer ist, das einzeln oder als Gemisch aufweist:

- mindestens eine Sequenz, die von einem linearen oder verzweigten, aliphatischen und/oder cycloaliphatischen und/oder aromatischen Polyester abgeleitet ist; und/oder
- mindestens eine Sequenz, die von einem aliphatischen und/oder cycloaliphatischen und/oder aromatischen Polyether abgeleitet ist; und/oder
- mindestens eine Siliconsequenz, die substituiert oder unsubstituiert ist, verzweigt oder unverzweigt vorliegt, beispielsweise Polydimethylsiloxan oder Polymethylphenylsiloxan; und/ oder
- mindestens eine Sequenz, die fluorierte Gruppen aufweist.

**75.** Zusammensetzung nach Anspruch 72, **dadurch gekennzeichnet, dass** das in der wässrigen Phase dispergierbare filmbildende Polymer unter den Polyestern, Polyesteramiden, Polyestern mit Fettkette, Polyamiden und Epoxyesterharzen ausgewählt ist.

**76.** Zusammensetzung nach Anspruch 72, **dadurch gekennzeichnet, dass** das in der wässrigen Phase dispergierbare filmbildende Polymer unter den Acrylpolymeren, Acrylcopolymeren und Vinylpolymere ausgewählt ist.

**77.** Zusammensetzung nach einem der vorhergehenden Ansprüche, **dadurch gekennzeichnet, dass** der Anteil des Filmbildners im Bereich von 2 bis 60 Gew.-%, vorzugsweise 5 bis 60 Gew.-% und noch bevorzugter 2 bis 30 Gew.-% der trockenen Verbindung, bezogen auf das Gesamtgewicht der Zusammensetzung, liegt.

**78.** Kosmetische Zusammensetzung nach einem der vorhergehenden Ansprüche, **dadurch gekennzeichnet, dass** sie ferner ein oder mehrere Farbmittel enthält, die unter den wasserlöslichen Farbstoffen und den pulverförmigen Farbmitteln, wie Pigmenten, Perlglanzpigmenten und Pailletten ausgewählt ist.

**79.** Kosmetische Zusammensetzung nach einem der vorhergehenden Ansprüche, **dadurch gekennzeichnet, dass**

sie als Suspension, Dispersion, Lösung, Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), als Creme, Paste, Schaum, Vesikeldispersion insbesondere von ionischen oder nichtionischen Lipiden, zweiphasige oder mehrphasige Lotion, Spray, Pulver, Paste, insbesondere weiche Paste oder wasserfreie Paste, Stick oder gegossener Feststoff vorliegt.

**80.** Zusammensetzung nach einem der vorhergehenden Ansprüche, **dadurch gekennzeichnet, dass** sie in wasserfreier Form vorliegt.

**81.** Kosmetische Zusammensetzung nach einem der vorhergehenden Ansprüche, **dadurch gekennzeichnet, dass** es sich um eine Zusammensetzung zum Schminken oder zur Pflege von Keratinsubstanzen handelt.

**82.** Zusammensetzung nach einem der vorhergehenden Ansprüche, **dadurch gekennzeichnet, dass** es sich um ein Produkt zum Schminken der Lippen handelt.

**83.** Zusammensetzung nach einem der vorhergehenden Ansprüche, **dadurch gekennzeichnet, dass** es sich um ein Produkt zum Schminken der Augen handelt.

**84.** Zusammensetzung nach einem der vorhergehenden Ansprüche, **dadurch gekennzeichnet, dass** es sich um ein Schminkprodukt für den Teint handelt.

**85.** Zusammensetzung nach einem der vorhergehenden Ansprüche, **dadurch gekennzeichnet, dass** es sich um ein Produkt zum Schminken der Nägel handelt.

**86.** Zusammensetzung zum Überzeihen von Keratinfasern und insbesondere der Wimpern und der Augenbrauen, die ein flüssiges, organisches Medium, mindestens eine wässrige Phase, mindestens ein filmbildendes, ethylenisches Sequenzpolymer nach einem der Ansprüche 1 bis 56 und mindestens einen weiteren, in der wässrigen Phase löslichen oder dispergierbaren Filmbildner enthält.

**87.** Zusammensetzung nach dem vorhergehenden Anspruch,
**dadurch gekennzeichnet, dass** der Filmbildner ein in der wässrigen Phase dispergierbares filmbildendes Polymer ist.

**88.** Zusammensetzung nach dem vorhergehenden Anspruch,
**dadurch gekennzeichnet, dass** das in der wässrigen Phase dispergierbare filmbildende Polymer unter den Polyurethanen, Polyurethan-acrylverbindungen, Polyurethanen-polyvinylpyrrolidonen, Polyester-polyurethanen, Polyether-polyurethanen, Polyharnstoffen, Polyharnstoff/ Polyurethanen und deren Gemischen ausgewählt ist.

**89.** Zusammensetzung nach einem der Ansprüche 86 bis 88,
**dadurch gekennzeichnet, dass** sie ein Wachs enthält.

**90.** Zusammensetzung nach einem der Ansprüche 86 bis 89,
**dadurch gekennzeichnet, dass** sie einen grenzflächenaktiven Stoff enthält.

**91.** Zusammensetzung nach einem der Ansprüche 86 bis 90,
**dadurch gekennzeichnet, dass** sie einen zweiten Filmbildner enthält, der unter den wasserlöslichen Polymeren ausgewählt ist.

**92.** Zusammensetzung nach dem vorhergehenden Anspruch,
**dadurch gekennzeichnet, dass** das oder die wasserlöslichen Polymere unter den kationischen Cellulosederivaten und/oder gegebenenfalls modifizierten Polymeren natürlicher Herkunft, wie Gummi arabicum, ausgewählt sind.

**93.** Zusammensetzung nach einem der Ansprüche 86 bis 92,
**dadurch gekennzeichnet, dass** sie ein Farbmittel enthält.

**94.** Zusammensetzung nach einem der Ansprüche 86 bis 93,
**dadurch gekennzeichnet, dass** es sich um Mascara handelt.

**95.** Kosmetische Einheit umfassend:

a) einen Behälter, der mindestens eine Abteilung abgrenzt, wobei der Behälter mit einer Verschlusseinrichtung verschlossen ist, und

b) eine Zusammensetzung, die sich im Inneren der Abteilung befindet, wobei die Zusammensetzung einer Zusammensetzung nach einem der vorhergehenden Ansprüche entspricht.

**96.** Kosmetische Einheit nach dem vorhergehenden Anspruch, **dadurch gekennzeichnet, dass** der Behälter zumindest zum Teil aus mindestens einem thermoplastischen Material gebildet ist.

**97.** Kosmetische Einheit nach Anspruch 95, **dadurch gekennzeichnet, dass** der Behälter zumindest zum Teil aus mindestens einem nichtthermoplastischen Material, insbesondere aus Glas oder Metall, gebildet ist.

**98.** Einheit nach einem der Ansprüche 95 bis 97, **dadurch gekennzeichnet, dass** in der geschlossenen Stellung des Behälters die Verschlusseinrichtung mit dem Behälter verschraubt ist.

**99.** Einheit nach einem der Ansprüche 95 bis 97, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten, Kleben oder Schweißen.

**100.** Einheit nach einem der Ansprüche 95 bis 99, **dadurch gekennzeichnet, dass** die Zusammensetzung im Inneren der Abteilung in etwa unter Atmosphärendruck steht.

**101.** Einheit nach einem der Ansprüche 95 bis 99, **dadurch gekennzeichnet, dass** die Zusammensetzung im Inneren des Behälters unter Druck steht.

**102.** Kosmetisches Verfahren zum Schminken oder für die Pflege von Keratinsubstanzen, das umfasst, eine kosmetische Zusammensetzung nach einem der Ansprüche 1 bis 94 auf die Keratinsubstanzen aufzutragen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6153206 A
- US 5725882 A
- US 5209924 A
- US 4972037 A
- US 4981903 A
- US 4981902 A
- US 5468477 A
- US 5219560 A
- EP 0388582 A
- US 5948393 A
- EP 0815836 A
- US 5849318 A
- FR 2232303 A
- FR 0113920
- EP 080976 A
- FR 2077143
- FR 2393573
- US 3589578 A
- US 4031307 A
- US 4131576 A
- US 4693935 A
- US 4728571 A
- EP 0412704 A
- EP 0412707 A
- EP 0640105 A
- WO 9500578 A
- EP 0582152 A
- WO 9323009 A
- WO 9503776 A
- US 5061481 A
- US 5849275 A
- US 6033650 A
- WO 9323446 A
- WO 9506078 A
- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618


### Littérature non-brevet citée dans la description


- Polymer Handbook. John Wiley, 1989
- Encyclopedia of Chemical Technology, KIRK-OTHMER. WILEY, 1979, vol. 22, 333-432